# EUROPÄISCHE PATENTSCHRIFT

(11) **EP 3 656 432 B1**
(45) Veröffentlichungstag und Bekanntmachung des Hinweises auf die Patenterteilung: **16.08.2023**
(21) Anmeldenummer: 19197034.2
(22) Anmeldetag: 12.09.2019
(51) Int. Cl.: A61M 25/00

(54) **VORRICHTUNG ZUR TEMPORÄREN, LOKALEN APPLIKATION VON FLUIDEN**
DEVICE FOR TEMPORARY LOCAL APPLICATION OF FLUIDS
DISPOSITIF D'APPLICATION TEMPORAIRE ET LOCALE DE FLUIDES

(30) Priorität: 29.10.2018 DE 102018218429
(43) Veröffentlichungstag der Anmeldung: 27.05.2020
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- US-A- 4 186 745
- US-A- 5 792 118
- US-A1- 2013 204 208
- US-A1- 2016 220 788

## Beschreibung

Die Erfindung betrifft eine medizinische Vorrichtung zur temporären, lokalen Applikation von Fluiden, insbesondere von pharmazeutischen Fluiden. Die Erfindung betrifft auch ein Schlauchsystem zum Aufbau einer solchen Vorrichtung, ein Verfahren zum Kürzen eines solchen Schlauchsystem oder einer solchen Vorrichtung und ein Verfahren zum Betreiben einer solchen Vorrichtung.

Die lokale Applikation von pharmazeutischen Wirkstoffen, insbesondere von Antibiotika, ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden. Das Einleiten von Fluiden in Hohlräume zum Zweck der Spülung und Desinfektion kann aber auch zur Desinfektion und Reinigung von medizinischen Geräten mit Hohlräumen nützlich sein, die ansonsten nur schwer zu erreichen wären.

Zur medizinischen Behandlung von Infektionen in schwer erreichbaren Hohlräumen und Kavitäten, wie Knochenhöhlen, sind resorbierbare und nichtresorbierbare Wirkstoffträger bekannt.

Exemplarisch für nichtresorbierbare Wirkstoffträger sind die seit 1977 unter dem Markennamen Septopal^{®} bekannten Kugelketten. Diese bestehen aus Polymethylmethacrylat-Kugeln, die das Breitbandantibiotikum Gentamicinsulfat enthalten, wobei diese Kugeln auf Stahlzwirn kettenförmig angeordnet sind (K. Klemm: Gentamicin-PMMA-beads in treating bone and soft tissue infections. Zentralbl. Chir. 104(14) (1979) 934-942.; K. Klemm: Antibiotic bead chains. Clin. Orthop. 295 (1993) 63-76.). Dieser kettenförmige Wirkstoffträger (Septopal^{®}) hat sich seit Jahrzehnten bei der lokalen antibiotischen Behandlung der Osteomyelits bewährt. Vorteilhaft ist dabei, dass das Gentamcinsulfat in größeren Mengen über einen Zeitraum von mehreren Tagen aus dem Wirkstoffträger freigesetzt wird. Vorteilhaft ist es weiterhin, dass der kettenförmige Wirkstoffträger vom medizinischen Anwender problemlos durch einfaches Abschneiden des Stahlzwirns mit überzähligen Kugeln an die anatomische Situation am Implantationsort angepasst werden kann. Nachteilig ist, dass der Wirkstoffträger ausschließlich Gentamicinsulfat enthält und dass der medizinische Anwender den Wirkstoffträger nicht mit weiteren Antibiotika, entsprechend der Empfindlichkeit der mikrobiellen Keime, modifizieren kann. Zudem kann die Abgabe des pharmazeutischen Wirkstoffs nicht mehr ohne Austausch der Kugelkette an den Verlauf der Behandlung angepasst werden, wenn die Kugelkette erst einmal implantiert ist. Dadurch ist insbesondere die erfolgreiche lokale Behandlung von Infektionen mit Problemkeimen, wie MRSA und VRSA, nur bedingt oder nicht möglich. Die Entfernung der Kugelketten nach erfolgter Wirkstofffreisetzung ist durch Verwachsung mit Bindegewebe für den Patienten mit einer erheblichen Belastung verbunden.

Exemplarisch für resorbierbare beziehungsweise biodegradierbare Wirkstoffträger sind Vliese und Schwämme aus Kollagen oder Gelatine. Exemplarisch seien dafür die Druckschriften DE 34 29 038 A1, DE 33 34 595 A1, DE 28 43 963 C2, DE 32 03 957 C2 und DE 33 34 595 A1 genannt. Diese enthalten Gentamicinsulfat oder Gemische aus Gentamicinsulfat und einem in Wasser gering löslichen Gentamicinsalz. Weiterhin gibt es eine Vielzahl von resorbierbaren beziehungsweise biodegradierbaren Wirkstoffträgern auf Basis von Tricalciumphosphat, Hydroxylapatit, Gips und deren Mischungen sowie auch Kompositmaterialien aus diesen Salzen und organischen Bindemitteln. Eine Übersicht wurde von Kühn et al. publiziert (K.-D. Kühn, N. Renz, A. Trampuz: Lokale Antibiotika-Therapie. Der Unfallchirurg. 120 (2017) 561-572).

Nachteilig an den aufgeführten nicht-resorbierbaren und auch den rersorbierbaren beziehungsweise biodegradierbaren Wirkstoffträgern ist, dass der antimikrobielle Wirkstoff durch die gewählte Zusammensetzung festgelegt ist und dass nach der Implantation des Wirkstoffträgers der Wirkstoff nicht mehr ausgetauscht oder mit anderen Wirkstoffen ergänzt werden kann. Weiterhin unterliegt die Wirkstofffreisetzung bei allen bisherigen lokalen Wirkstofffreisetzungssystemen auf dem Prinzip der Diffusion, so dass hohe Wirkstoffmengen nur in den ersten Stunden oder maximal Tagen freigesetzt werden. Eine Ausnahme bildet die Verwendung von in Wasser gering löslichen Wirkstoffsalzen, bei denen die Wirkstofffreisetzung vom Löslichkeitsgleichgewicht der Wirkstoffsalze abhängt.

US 5 792 118 A beschreibt einen Katheter als einen äußeren Schlauch mit einem selektiv aufblasbaren Ballonventil als einen inneren Schlauch, um eine oder mehrere in der Katheterwand befindliche durchgehenden Öffnungen zu verschließen.

US 2016/220788 A1 beschreibt einen Katheter, der in der Lage ist, die Länge eines Infusionsabschnitts in situ stufenlos einzustellen, um eine effektive Infusionslänge und Infusionsgeschwindigkeit zu steuern.

Wünschenswert ist daher ein Wirkstoffträger, der eine lokale Applikation beliebiger pharmazeutischer Wirkstoffe zulässt und wobei der pharmazeutische Wirkstoff jederzeit gegen andere fluide pharmazeutische Wirkstoffe ausgetauscht werden kann. Außerdem ist es wünschenswert, dass die Wirkstoffkonzentration, die unmittelbar am Implantationsort erreicht wird, direkt von außen eingestellt werden kann.

Der Erfindung liegt die Aufgabe zugrunde, eine medizinische Vorrichtung zur lokalen Applikation von Fluiden bereitzustellen, mit der eine lokale und temporäre Abgabe des Fluids in schwer zugänglichen Bereichen ermöglicht wird. Die Vorrichtung soll dabei flexibel an unterschiedliche Anwendungsbereiche anzupassen sein. Bei der Verwendung zur Behandlung einer Infektion soll eine möglichst schonende Behandlung möglich sein, bei der das angrenzende entzündete Gewebe möglichst wenig gereizt wird und zwar sowohl bei einer temporären Abgabe des Fluids als auch beim Einsetzen und Entfernen des eingeführten Teils der Vorrichtung. Die Vorrichtung soll auch über längere Zeiträume an einem bestimmten Ort zur wiederholten Abgabe des Fluids geeignet sein, ohne dass die Vorrichtung hierfür entfernt werden muss. Die Vorrichtung soll kostengünstig zu fertigen sein und möglichst ein hygienisches, nur einmal verwendbares Wegwerfprodukt sein. Dabei soll zumindest der in den zu spülenden Hohlraum platzierbare Teil der Vorrichtung oder eben die gesamte Vorrichtung als Wegwerfprodukt kostengünstig und leicht entsorgbar sein.

Die Aufgabe der Erfindung besteht somit auch darin, eine einfache, kostengünstige Vorrichtung zur lokalen Applikation von Fluiden zu entwickeln. Die Vorrichtung soll eine lokale Applikation von pharmazeutischen Fluiden mit beliebiger Zusammensetzung, zum Beispiel antibiotischen Lösungen, ermöglichen. Ein Teil der Vorrichtung befindet sich bei einer medizinischen Anwendung nach einer Implantation im Patienten und ein zweiter Teil der Vorrichtung außerhalb des Patienten. Die pharmazeutischen Fluide sollen in dem außerhalb des im Patienten befindlichen Teils der Vorrichtung eingebracht werden können und zum Implantationsort durch die Vorrichtung geleitet und dort freigesetzt werden. Die Vorrichtung soll plastisch verformbar sein, um den anatomischen Gegebenheiten am Implantationsort oder der geometrischen Form der Hohlform folgen zu können. Die Freisetzung von pharmazeutischen Fluiden soll aus längs an der Vorrichtung angeordneten Öffnungen erfolgen. Die Öffnungen sollen idealerweise reversibel verschließbar sein, um ein Einwachsen von Bindegewebe beziehungsweise eine Verstopfung der Öffnungen durch koaguliertes Blut zu verhindern. Weiterhin soll die Vorrichtung so beschaffen sein, dass der im Patienten befindliche Teil der Vorrichtung durch Kürzen der Länge an die jeweilige anatomische Situation des Patienten angepasst werden kann, ohne dass die Funktion der Vorrichtung beeinträchtigt wird.

Die Aufgaben der Erfindung werden gelöst durch eine medizinische Vorrichtung zur lokalen Applikation eines Fluids aufweisend
A) einen flexibel verformbaren äußeren Schlauch mit einer Schlauchwand, wobei der äußere Schlauch mehrere durchgehende Öffnungen in der Schlauchwand aufweist, wobei zumindest eine der mehreren durchgehenden Öffnungen im Bereich eines ersten Endes des äußeren Schlauchs angeordnet ist,
B) einen flexiblen inneren Schlauch, wobei der innere Schlauch zumindest bereichsweise im Inneren des äußeren Schlauchs angeordnet ist, wobei der innere Schlauch in einem expandierten Zustand einen Außendurchmesser hat, der zumindest genauso groß ist, wie der Innendurchmesser des äußeren Schlauchs, so dass der innere Schlauch die durchgehenden Öffnungen des äußeren Schlauchs im expandierten Zustand an der Innenseite des äußeren Schlauchs verschließt, wobei der Außendurchmesser des inneren Schlauchs von dem expandierten Zustand in einen radial kontrahierten Zustand mit geringerem Außendurchmesser des inneren Schlauchs überführbar ist, so dass die Öffnungen freigelegt sind,
C) eine Verbindung, die den äußeren Schlauch und den inneren Schlauch am ersten Ende des äußeren Schlauchs fest miteinander verbindet und fluiddicht verschließt, und
D) einen Konnektor zum Einspeisen des Fluids in den Zwischenraum zwischen der Innenseite des äußeren Schlauchs und der Außenseite des inneren Schlauchs, wobei der Konnektor im Bereich eines zweiten Endes des äußeren Schlauchs angeordnet ist, wobei das zweite Ende des äußeren Schlauchs dem ersten Ende des äußeren Schlauchs gegenüberliegend angeordnet ist, wobei der expandierte Zustand der entspannte Zustand des inneren Schlauchs ist und durch eine Krafteinwirkung in den radial kontrahierten Zustand überführbar ist.

Mit der Vorrichtung können auch medizinische Instrumente abgespült oder ausgespült werden, insbesondere medizinischen Instrumente mit Hohlräumen, in die der äußere Schlauch eingeführt werden kann. Die Vorrichtung kann aber auch zum freien Verteilen des Fluids verwendet werden. Besonders geeignet ist jedoch eine medizinische Anwendung der erfindungsgemäßen Vorrichtung, bei der der äußere Schlauch in eine Kavität eines menschlichen Körpers eingeführt wird und das Fluid zur Behandlung des angrenzenden Gewebes eingesetzt wird.

Es sind mindestens zwei oder mehr durchgehende Öffnungen in der seitlichen Schlauchwand des äußeren Schlauchs angeordnet. Dadurch kann der äußere Schlauch mit dem inneren Schlauch auch bei Entfernung einer der durchgehenden Öffnungen wenigstens einmal gekürzt werden, ohne dass nach dem Kürzen keine der verbliebenen Öffnungen mehr freiliegt.

Der äußere Schlauch ist an dem ersten Ende des äußeren Schlauchs fluiddicht verschlossen.

Die durchgehenden Öffnungen verbinden das Innere des äußeren Schlauchs mit der äußeren Umgebung des äußeren Schlauchs.

Die Schlauchwand des äußeren Schlauchs ist die alle Seiten umgebende seitliche Wandung des äußeren Schlauchs.

Die seitliche Schlauchwand ist die Mantelfläche des äußeren Schlauchs und auch des inneren Schlauchs. Die seitliche Schlauchwand ist damit der gesamte Mantel, der die beiden Enden (das erste Ende und das zweite Ende) des jeweiligen Schlauchs verbindet. Bei geraden Schläuchen mit einer zylindrischen Geometrie ist die Mantelfläche die Wandung senkrecht zur Zylinderachse des zylindrischen Schlauchs. Die durchgehenden Öffnungen befinden sich also in der Mantelfläche.

Bevorzugt ist vorgesehen, dass der innere Schlauch den äußeren Schlauch am ersten Ende des äußeren Schlauchs verschließt.

Vorzugsweise ist zumindest eine der mehreren durchgehenden Öffnungen in einem Abstand von maximal 1,5 cm zum ersten Ende des äußeren Schlauchs angeordnet. Besonders bevorzugt ist zumindest eine der mehreren durchgehenden Öffnungen in einem Abstand von maximal 5 mm zum ersten Ende des äußeren Schlauchs angeordnet. Dadurch kann sichergestellt werden, dass auch der Teil des zu spülenden Hohlraums, an den die Spitze mit dem ersten Ende des äußeren Schlauchs angeordnet ist, von dem Fluid erreicht werden kann und somit einer Spülwirkung oder einer medizinischen Behandlung zugänglich ist.

Vorzugsweise ist der Konnektor in einem Abstand von maximal 5 cm zum zweiten Ende des äußeren Schlauchs angeordnet. Besonders bevorzugt ist der Konnektor in einem Abstand von maximal 2 mm zum zweiten Ende des äußeren Schlauchs angeordnet. Dadurch muss die Vorrichtung nicht übermäßig lang aufgebaut werden und der Konnektor ist auch dann gut von außen zugänglich, wenn der äußere Schlauch tief in eine Kavität oder einen Hohlraum eingeführt ist.

Der innere Schlauch ist vorzugsweise radial verformbar. Dadurch kann er von dem kontrahierten Zustand in den expandierten Zustand überführt werden, ohne dass ein Faltenwurf entsteht.

Das Fluid ist durch den Konnektor und durch den Zwischenraum zwischen der Innenseite des äußeren Schlauchs und der Außenseite des inneren Schlauchs durch die mehreren durchgehenden Öffnungen im äußeren Schlauch applizierbar, wenn der innere Schlauch sich im kontrahierten Zustand befindet. Sobald sich der innere Schlauch also im kontrahierten Zustand befindet, existiert eine für das Fluid durchlässige Verbindung, in Form eines Zwischenraums zwischen den Schläuchen, zwischen dem Konnektor und den durchgehenden Öffnungen, durch die das Fluid appliziert werden kann.

Der Konnektor kann ein als T-Stück geformter Anschluss am äußeren Schlauch oder an an einem damit verbundenen Hohlraum sein, kann aber auch ganz einfach durch das zweite Ende des äußeren Schlauchs realisiert werden, durch das das Fluid eingespeist werden kann.

Der innere Schlauch muss durch die Wandung des äußeren Schlauchs im Bereich des zweiten Endes des äußeren Schlauchs oder durch einen Abschluss des äußeren Schlauchs am zweiten Ende des äußeren Schlauchs hindurchgeführt sein.

Der expandierte Zustand ist der entspannte Zustand des inneren Schlauchs, während der radial kontrahierte Zustand des inneren Schlauchs durch eine Krafteinwirkung, wie einen Unterdruck oder einen Sog, im radial kontrahierten Zustand gehalten werden muss. Dies hat den Vorteil, dass die durchgehenden Öffnungen im äußeren Schlauch im entspannten Zustand geschlossen sind. Dadurch können die Öffnungen nicht versehentlich geöffnet bleiben und sich nicht öffnen, wenn sich der innere Schlauch aufgrund nachlassender (innerer) Kräfte entspannt.

Das erste Ende des äußeren Schlauchs kann auch als das distale Ende des äußeren Schlauchs bezeichnet werden. Das erste Ende des äußeren Schlauchs definiert der Einfachheit halber auch das erste Ende (dann auch das distale Ende) des inneren Schlauchs. Das zweite Ende des äußeren Schlauchs, das dem Konnektor und dem Anwender zugewandt ist, ist dementsprechend das proximale Ende des äußeren Schlauchs.

Es kann erfindungsgemäß vorgesehen sein, dass der innere Schlauch durch Erzeugen eines Unterdrucks im Inneren des inneren Schlauchs oder durch eine Volumenreduktion einer Flüssigkeit im Inneren des inneren Schlauchs in den radial kontrahierten Zustand überführbar ist.

Hierdurch können durch Erzeugen des Unterdrucks oder der Volumenreduktion im inneren Schlauch die mehreren durchgehenden Öffnungen im äußeren Schlauch zuverlässig mechanisch geöffnet werden. Zudem schließen sich die durchgehenden Öffnungen im äußeren Schlauch auch selbstständig wieder, wenn der innere Schlauch sich wieder in den expandierten Zustand ausdehnt. Die durchgehenden Öffnungen im äußeren Schlauch können so leicht und schnell freigelegt werden. Insbesondere wird die äußere Form des äußeren Schlauchs beim Öffnen der durchgehenden Öffnungen nicht verändert und nicht verformt, so dass eine Irritation des angrenzenden Gewebes durch den Öffnungsvorgang minimiert wird.

Des Weiteren kann vorgesehen sein, dass der innere Schlauch durch einen Verschluss des äußeren Schlauchs am zweiten Ende des äußeren Schlauchs oder durch die seitliche Schlauchwand des äußeren Schlauchs im Bereich des zweiten Endes geführt ist.

Hierdurch ist der innere Schlauch von außen zugänglich, so dass dadurch der Druck im Inneren des inneren Schlauchs beziehungsweise das Volumen des Inneren Schlauch von außen beeinflusst werden kann, um den inneren Schlauch vom expandierten in den radial kontrahierten Zustand zu überführen und umgekehrt.

Dabei kann vorgesehen sein, dass eine Bedieneinrichtung an einem durch den Verschluss oder die Schlauchwand des äußeren Schlauchs geführten Ende des inneren Schlauchs befestigt ist, wobei mit der Bedieneinrichtung der Druck eines Gases innerhalb des inneren Schlauchs veränderbar ist oder das Volumen einer Flüssigkeit innerhalb des inneren Schlauchs veränderbar ist und durch eine Reduzierung des Drucks des Gases oder des Volumens der Flüssigkeit der innere Schlauch vom expandierten in den radial kontrahierten Zustand überführbar ist.

Hierdurch kann auf einfache Weise mechanisch der expandierte Zustand und der radial kontrahierte Zustand des inneren Schlauchs eingestellt werden.

Bei einer Weiterbildung der vorliegenden Erfindung kann vorgesehen sein, dass die Vorrichtung eine Bedieneinrichtung aufweist, insbesondere eine Kolbenspritze, eine Pumpe oder einen Peleusball als Bedieneinrichtung aufweist, mit der der innere Schlauch innerhalb des äußeren Schlauchs von dem expandierten Zustand in den radial kontrahierten Zustand überführbar ist, so dass die Öffnungen im äußeren Schlauch geöffnet werden.

Hierdurch kann die Vorrichtung auf einfache Weise von außen bedient werden, das heißt, der innere Schlauch vom expandierten Zustand in den radial kontrahierten Zustand überführt werden und vom radial kontrahierten Zustand in den expandierten Zustand überführt werden.

Kolbenspritzen sind dabei erfindungsgemäß bevorzugt. Bei der Verwendung einer Kolbenspritze als Bedieneinrichtung ist vorzugsweise vorgesehen, dass ein Druckkolben der Kolbenspritze mit einer lösbaren Arretierung arretierbar ist.

Bevorzugt kann vorgesehen sein, dass die Verbindung durch ein Verbindungselement realisiert ist.

Hierdurch kann eine variable Verbindung verwendet werden, da das Verbindungselement neu positioniert und an einem neu hergestellten distalen ersten Ende der Schläuche befestigt werden kann, nachdem die Schläuche gekürzt wurden.

Dabei kann vorgesehen sein, dass das Verbindungselement einen konischen oder zylindrischen Vorsprung aufweist, der in den inneren Schlauch gesteckt oder geschraubt ist, so dass der innere Schlauch im Bereich des ersten Endes des äußeren Schlauchs durch den Vorsprung derart geweitet ist, dass der innere Schlauch den äußeren Schlauch am ersten Ende des äußeren Schlauchs fluiddicht verschließt.

Hiermit lassen sich die distalen (ersten) Enden der Schläuche zuverlässig abdichten und verbinden.

Ferner kann dabei vorgesehen sein, dass das Verbindungselement lösbar mit dem ersten Ende des äußeren Schlauchs und/oder dem inneren Schlauch verbunden ist.

Durch diese beiden Maßnahmen kann eine variable Verbindung verwendet werden, so dass der Schlauch bei verschiedenen Längen gekürzt werden kann und die Verbindung auf einfache Weise durch Einstecken oder Einschrauben des Verbindungselements vom Anwender erzeugt werden kann. Hierdurch kann die Vorrichtung vom Anwender selbst an unterschiedliche Einsatzgebiete oder Behandlungssituationen angepasst werden.

Es kann auch vorgesehen sein, dass das Verbindungselement einen ersten rotationssymmetrischen Körper mit einem Außengewinde oder mit Stegen an der Außenseite aufweist, wobei das Außengewinde oder der rotationssymmetrische Körper einen größeren Außendurchmesser aufweist als der Innendurchmesser des inneren Schlauchs.

Hierdurch kann die Verbindung nach einem Kürzen der Schläuche neu positioniert werden und dabei das erste Ende des äußeren Schlauchs und der innere Schlauch an dieser Stelle mit dem Verbindungselement abgedichtet werden.

Dabei kann vorgesehen sein, dass das Verbindungselement einen zweiten rotationssymmetrischen Körper mit einem Außendurchmesser aufweist, der kleiner oder gleich dem Außendurchmesser des äußeren Schlauchs ist, wobei der erste rotationssymmetrische Körper und der zweite rotationssymmetrische Körper axial miteinander verbunden sind und wobei bevorzugt der zweite rotationssymmetrische Körper in axialer Richtung des Verbindungselements wenigstens 5 mm lang ist.

Hierdurch wird sichergestellt, dass das Verbindungselement manuell greifbar und zur Herstellung der Verbindung manuell einsteckbar oder einschraubbar ist.

Gemäß einer bevorzugten Weiterbildung kann vorgesehen sein, dass das Verbindungselement in den inneren Schlauch auf der Seite des ersten Endes des äußeren Schlauchs eingeschraubt oder eingepresst ist und dabei den gesamten freien Querschnitt des inneren Schlauchs vollständig flüssigkeitsdicht oder flüssigkeitsdicht und gasdicht verschließt, wobei der so verschlossene innere Schlauch dadurch derart gegen die Innenseite des äußeren Schlauchs gepresst ist, dass der äußere Schlauch am ersten Ende durch den inneren Schlauch flüssigkeitsdicht und gasdicht verschlossen ist.

Hierdurch wird erreicht, dass beide Schlauchenden am ersten Ende des äußeren Schlauchs auf einfache Weise mit dem Verbindungselement verschlossen werden und dabei die Verbindung realisieren. Dadurch können der innere und der äußere Schlauch gekürzt werden, wobei das Verbindungselement durch Einschrauben oder Einpressen in das durch das Abschneiden entstandene neue Ende verschlossen wird. Hierdurch ist die Vorrichtung auf einfache Weise an die jeweilige Behandlungssituation anpassbar. Dadurch, dass der äußere Schlauch am ersten Ende durch den inneren Schlauch flüssigkeitsdicht und gasdicht verschlossen ist, wird mit dem Verbindungselement erreicht, dass kein Hohlraum zwischen der Außenseite des inneren Schlauchs und der Innenseite des äußeren Schlauchs entlang der axialen Erstreckung des Verbindungselements vorhanden ist.

Es kann des Weiteren vorgesehen sein, dass der Zwischenraum zwischen dem äußeren Schlauch und dem inneren Schlauch am zweiten Ende des äußeren Schlauchs fluiddicht verschlossen ist.

Hierdurch wird vermieden, dass das Fluid an anderen Stellen außer durch die durchgehenden Öffnungen austreten kann. Der Zwischenraum zwischen dem äußeren Schlauch und dem inneren Schlauch ist dann bis auf die durchgehenden Öffnungen in der Schlauchwand des äußeren Schlauchs und bis auf den Konnektor nach außen fluiddicht verschlossen. Das Fluid kann dann nur noch durch den Konnektor eingespeist und durch die durchgehenden Öffnungen abgegeben werden, wenn letztere geöffnet sind.

Es wird ferner vorgeschlagen, dass der äußere Schlauch an seinem ersten Ende den inneren Schlauch umschließt.

Hierdurch kann der innere Schlauch zum Abdichten des äußeren Schlauchs am ersten Ende des äußeren Schlauchs verwendet werden. Bevorzugt umschließt der äußere Schlauch auch an seinem zweiten Ende den inneren Schlauch.

Es kann vorgesehen sein, dass ein Gas in dem inneren Schlauch enthalten ist, wobei der innere Schlauch durch Reduzieren des Drucks des Gases im inneren Schlauch in den radial kontrahierten Zustand überführbar ist und durch erneutes Einleiten des Gases oder eines Gases wieder in den expandierten Zustand überführbar ist, oder eine Flüssigkeit den inneren Schlauch füllt, wobei der innere Schlauch durch Absaugen eines Teils der Flüssigkeit aus dem inneren Schlauch in den radial kontrahierten Zustand überführbar ist und durch erneutes Einleiten der Flüssigkeit oder anderer Flüssigkeit in den inneren Schlauch wieder in den expandierten Zustand überführbar ist.

Auf diese Weise kann durch Absaugen und Eindrücken eines Gases oder einer Flüssigkeit aus dem beziehungsweise in den inneren Schlauch der expandierte Zustand und der radial kontrahierte Zustand eingestellt werden und damit die mehreren durchgehenden Öffnungen im äußeren Schlauch geöffnet und geschlossen werden. Theoretisch kann auch eine Mischung aus Gas und Flüssigkeit im Inneren des inneren Schlauchs enthalten sein und zum Einstellen des expandierten und des radial kontrahierten Zustands verwendet werden.

Es kann vorgesehen sein, dass an dem inneren Schlauch eine Bedieneinrichtung zum Absaugen des Gases oder der Flüssigkeit aus dem inneren Schlauch angeschlossen ist, so dass der innere Schlauch durch Absaugen des Gases oder der Flüssigkeit in den radial kontrahierten Zustand überführbar ist und sich dadurch die Öffnungen im äußeren Schlauch öffnen.

Die Bedieneinrichtung zum Absaugen des Gases oder der Flüssigkeit kann vorzugsweise eine Einrichtung zum Erzeugen eines Unterdrucks sein, wie beispielsweise ein in einem Hubraum axial beweglicher Druckkolben, eine Kolbenspritze, eine Pumpe oder Peleusball.

Hiermit wird die "Bedienung" des inneren Schlauchs beziehungsweise das Öffnen und Schließen der durchgehenden Öffnungen vereinfacht.

Erfindungsgemäße Vorrichtungen können sich auch dadurch auszeichnen, dass die Innenseite des äußeren Schlauchs mit der Außenseite des inneren Schlauchs ein Ventil bildet.

Hierdurch wird klargestellt, dass die durchgehenden Öffnungen in dem äußeren Schlauch vom inneren Schlauch vollständig geschlossen werden können.

Dabei kann vorgesehen sein, dass das Ventil durch Reduzieren eines Gasdrucks im Inneren des inneren Schlauchs oder durch Absaugen einer Flüssigkeit aus dem Inneren des inneren Schlauchs und eine daraus resultierende radiale Kontraktion des inneren Schlauchs zu öffnen ist.

Hiermit wird eine einfache Möglichkeit zur Bedienung des Ventils mit Hilfe einer Druckveränderung im inneren Schlauch gegeben.

Ferner kann vorgesehen sein, dass zumindest eines der Elemente ausgewählt aus der Verbindung, dem äußeren Schlauch und dem inneren Schlauch ein röntgenopakes Material aufweist oder aus einem röntgenopaken Material besteht, wobei bevorzugt das röntgenopake Material ausgewählt ist aus Edelstahl, Titan, Titanlegierungen, Tantal, Tantallegierungen, Bariumsulfat enthaltenden Kunststoff und Zirkoniumdioxid enthaltenden Kunststoff oder Kombinationen daraus.

Hierdurch kann die Lage des äußeren Schlauchs beziehungsweise des inneren Schlauchs und gegebenenfalls der Verbindung im Körper eines Patienten bei Röntgenaufnahmen leicht erkannt werden. Hierzu ist es im Falle des äußeren und des inneren Schlauchs vorteilhaft, wenn der Röntgenopaker entlang der Längsachse des jeweiligen Schlauchs verteilt beziehungsweise angeordnet ist. Selbstverständlich wird das Verbindungselement sofern vorhanden aus oder mit einem der Materialien Edelstahl, Titan, Titanlegierungen, Tantal, Tantallegierung(en) oder Kombinationen daraus aufgebaut, während der innere Schlauch und/oder der äußere Schlauch hauptsächlich aus oder mit Bariumsulfat enthaltenden Kunststoff und Zirkoniumdioxid enthaltenden Kunststoff oder Kombinationen daraus aufgebaut wird.

Ferner kann vorgesehen sein, dass die mehreren durchgehenden Öffnungen alle, paarweise oder gruppenweise in axialer Richtung des äußeren Schlauchs zueinander beabstandet sind.

Hierdurch kann das Fluid an verschiedenen axial voneinander beabstandeten Stellen austreten. Zudem können der äußere und der innere Schlauch an verschiedenen Stellen in der Länge gekürzt werden, wobei gleichzeitig immer noch wenigstens eine der mehreren durchgehenden Öffnungen in dem äußeren Schlauch vorhanden sind.

Des Weiteren kann vorgesehen sein, dass im Inneren des inneren Schlauchs Wasser, eine physiologische Kochsalzlösung oder eine Ringer-Lösung oder Luft angeordnet ist, wobei durch eine Verringerung des Drucks der Luft oder des Volumens des Wassers, der physiologischen Kochsalzlösung oder der Ringer-Lösung der innere Schlauch in den radial kontrahierten Zustand überführbar ist.

Hiermit können die durchgehenden Öffnungen auf einfache Weise geöffnet werden.

Gemäß einer besonders bevorzugten Weiterbildung kann vorgesehen sein, dass ein Indikator mit dem inneren Schlauch verbunden ist, an dem von außerhalb der Vorrichtung ablesbar ist, ob sich der innere Schlauch im expandierten Zustand oder im kontrahierten Zustand befindet, wobei vorzugsweise der Indikator ein Unterdruckindikator ist, der mit dem inneren Schlauch gasdurchlässig verbunden ist.

Hierdurch kann der Anwender auf einfache Weise erkennen und feststellen, ob sich der innere Schlauch im radial kontrahierten Zustand befindet oder nicht und damit ob die durchgehenden Öffnungen im äußeren Schlauch offen oder geschlossen sind.

Zur Erhaltung der Elastizität des inneren Schlauchs kann vorgesehen sein, dass in der Wandung des inneren Schlauchs zumindest ein Metalldraht oder zumindest eine Metallspirale angeordnet ist, wobei der zumindest eine Metalldraht oder die zumindest eine Metallspirale bevorzugt entlang der gesamten Länge des inneren Schlauchs angeordnet sind.

Hiermit wird die Elastizität des inneren Schlauchs auch bei mehrmals wiederholten Überführungen des inneren Schlauchs in den radial kontrahierten Zustand sichergestellt. Dadurch wird verhindert, dass auch bei sehr häufiger Kontraktion des inneren Schlauchs sich dieser anschließend wieder ausdehnen kann, um die durchgehenden Öffnungen im äußeren Schlauch wieder zu verschließen.

Zur Vermeidung einer Verunreinigung des Fluids kann vorgesehen sein, dass in dem Konnektor oder in einer Verbindung des Konnektors zu dem Zwischenraum zwischen dem äußeren Schlauch und dem inneren Schlauch ein Rückschlagventil angeordnet ist, das ein Fließen des Fluids aus dem Zwischenraum heraus in den Konnektor hinein oder aus dem Konnektor heraus verhindert.

Hierdurch wird verhindert, dass gebrauchtes Fluid in ein an den Konnektor angeschlossenes Reservoir des Fluids zurückströmen kann und dadurch das Reservoir verunreinigt oder kontaminiert.

Bevorzugt kann vorgesehen sein, dass der innere Schlauch zumindest bereichsweise koaxial im Inneren des äußeren Schlauchs angeordnet ist.

Der innere Schlauch kann alternativ auch entlang einer Verbindungslinie punktuell oder durchgehend fest mit dem äußeren Schlauch verbunden sein. Bevorzugt ist der innere Schlauch jedoch nicht fest mit dem äußeren Schlauch verbunden. Durch eine koaxiale Anordnung lassen sich durchgehende Öffnungen am gesamten Umfang des äußeren Schlauchs realisieren. Zudem wird so die Montage der Vorrichtung vereinfacht.

Zur Aufrechterhaltung des Drucks des Fluids im gesamten Zwischenraum zwischen dem äußeren Schlauch und dem inneren Schlauch kann vorgesehen sein, dass die Summe der freien Querschnitte der durchgehenden Öffnungen im äußeren Schlauch gleich oder kleiner ist als der Querschnitt des Zwischenraums zwischen der Innenwand des äußeren Schlauchs und der Außenwand des inneren Schlauchs im radial kontrahierten Zustand.

Dadurch ist es möglich, dass aus allen Öffnungen entlang des äußeren Schlauchs das Fluid austreten kann, wenn der Zwischenraum zwischen der Innenwand des äußeren Schlauchs und der Außenwand des inneren Schlauchs mit Druck beaufschlagt wird.

Die der vorliegenden Erfindung zugrundeliegenden Aufgaben werden auch gelöst durch ein Schlauchsystem zum Aufbau einer erfindungsgemäßen Vorrichtung, aufweisend einen flexibel verformbaren äußeren Schlauch mit einer Schlauchwand, wobei der äußere Schlauch mehrere durchgehende Öffnungen in der Schlauchwand aufweist, und einen flexiblen inneren Schlauch, wobei der innere Schlauch zumindest bereichsweise im Inneren des äußeren Schlauchs angeordnet ist, wobei der innere Schlauch in einem expandierten Zustand einen Außendurchmesser hat, der zumindest genauso groß ist, wie der Innendurchmesser des äußeren Schlauchs, so dass der innere Schlauch die durchgehenden Öffnungen des äußeren Schlauchs im expandierten Zustand an der Innenseite des äußeren Schlauchs verschließt, wobei der Außendurchmesser des inneren Schlauchs von dem expandierten Zustand in einen radial kontrahierten Zustand mit geringerem Außendurchmesser des inneren Schlauchs überführbar ist, so dass die Öffnungen freigelegt sind, wobei der expandierte Zustand der entspannte Zustand des inneren Schlauchs ist und durch eine Krafteinwirkung in den radial kontrahierten Zustand überführbar ist.

Das Schlauchsystem kann zum Aufbau der erfindungsgemäßen Vorrichtung verwendet werden, indem es mit den anderen Elementen verbunden wird und die Verbindung zwischen dem äußeren und dem inneren Schlauch an einem ersten Ende des äußeren Schlauchs hergestellt wird.

Weiterhin wird exemplarisch ein Verfahren zum Kürzen und Verschließen eines solchen Schlauchsystems oder des inneren Schlauchs und des äußeren Schlauchs einer erfindungsgemäßen Vorrichtung beschrieben, gekennzeichnet durch Kürzen, insbesondere Abschneiden, des äußeren Schlauchs und des inneren Schlauchs ausgehend von einem ursprünglichen ersten Ende des äußeren Schlauchs, so dass diese ein neues gemeinsames erstes Ende bilden, und anschließendes Einschrauben oder Einpressen eines Verbindungselements in das offene erste Ende des inneren Schlauchs am ersten Ende des Schlauchs, so dass der gesamte freie Querschnitt des inneren Schlauchs vollständig flüssigkeitsdicht oder flüssigkeitsdicht und gasdicht verschlossen wird, wobei der so verschlossene innere Schlauch dadurch flächig gegen die Innenseite des äußeren Schlauchs gepresst wird, so dass der äußere Schlauch am ersten Ende durch den inneren Schlauch flüssigkeitsdicht und gasdicht verschlossen wird. Dieses Verfahren ist nicht Teil der beanspruchten Erfindung.

Hierdurch wird sichergestellt, dass das Fluid nicht an dem ersten Ende des äußeren Schlauchs austreten kann und dass der Druck im inneren Schlauch oder das Volumen des inneren Schlauchs veränderbar ist.

Das Einschrauben oder Einpressen des Verbindungselements erfolgt bevorzugt manuell.

Es kann ausreichen, wenn nach dem Kürzen des äußeren Schlauchs auch nur eine durchgehende Öffnung der mehreren durchgehenden Öffnungen im äußeren Schlauch verbleibt, die durch den Zwischenraum zwischen dem inneren Schlauch und dem äußeren Schlauch für das Fluid zugänglich ist.

Die der vorliegenden Erfindung zugrundeliegenden Aufgaben werden auch gelöst durch ein Verfahren zum Betreiben einer erfindungsgemäßen Vorrichtung umfassend die folgenden Schritte
A) Überführen des inneren Schlauchs vom expandierten Zustand in den kontrahierten Zustand, wobei dadurch die Öffnungen in dem äußeren Schlauch geöffnet werden; und
B) Einleiten des Fluids in den Zwischenraum zwischen dem äußeren Schlauch und dem inneren Schlauch.

Dabei kann vorgesehen sein, dass das Überführen des inneren Schlauchs vom expandierten Zustand in den kontrahierten Zustand in Schritt A) dadurch erfolgt, dass der Druck eines Gases im Inneren des inneren Schlauchs reduziert wird oder das Volumen einer Flüssigkeit im Inneren des inneren Schlauchs reduziert wird, wobei dadurch der Außendurchmesser des inneren Schlauchs verringert wird und dadurch die mehreren Öffnungen in der Schlauchwandung des äußeren Schlauchs freigelegt werden.

Hierdurch kann der Wechsel zwischen dem kontrahierten Zustand und dem expandierten Zustand auf einfache und zuverlässige Weise erfolgen.

Dabei kann wiederum vorgesehen sein, dass nach Schritt B) das Gas oder ein Gas oder die Flüssigkeit oder eine Flüssigkeit in den inneren Schlauch eingeleitet wird und dadurch der innere Schlauch expandiert und dadurch die mehreren Öffnungen des äußeren Schlauchs wieder verschlossen werden.

Hierdurch ist das Verfahren zum zeitweisen Einleiten oder Ausströmen des Fluids geeignet.

Es kann auch vorgesehen sein, dass vor Schritt A) ein Verfahren zum Kürzen und Verschließen eines erfindungsgemäßen Schlauchsystems oder des inneren Schlauchs und des äußeren Schlauchs einer erfindungsgemäßen Vorrichtung durchgeführt wird, bei dem, durch Kürzen, insbesondere Abschneiden, des äußeren Schlauchs und des inneren Schlauchs ausgehend von einem ursprünglichen ersten Ende des äußeren Schlauchs, so dass diese ein gemeinsames neues erstes Ende bilden, und anschließendes Einschrauben oder Einpressen eines Verbindungselements in das offene erste Ende des inneren Schlauchs am ersten Ende des Schlauchs, so dass der gesamte freie Querschnitt des inneren Schlauchs vollständig flüssigkeitsdicht oder flüssigkeitsdicht und gasdicht verschlossen wird, wobei der so verschlossene innere Schlauch dadurch flächig gegen die Innenseite des äußeren Schlauchs gepresst wird, so dass der äußere Schlauch am ersten Ende durch den inneren Schlauch flüssigkeitsdicht und gasdicht verschlossen wird.

Der Erfindung liegt die überraschende Erkenntnis zugrunde, dass es mit Hilfe eines im radialen Umfang veränderbaren inneren Schlauchs gelingt, die in der Schlauchwandung eines äußeren Schlauchs vorhandenen Öffnungen reversibel zu öffnen und zu schließen, um so temporär ein Fluid abzugeben. Durch einfaches Ausdehnen und Zusammenziehen des inneren Schlauchs können die Öffnungen verschlossen oder geöffnet werden. Gleichzeitig werden dabei auf den äußeren Schlauch keine Kräfte ausgeübt, die zu einer starken Vorformung des äußeren Schlauchs führen. Dadurch bleibt der äußere Schlauch formstabil. Eine mechanische Beanspruchung der angrenzenden mit dem Fluid zu behandelnden Oberflächen wird so vermieden. Das hierdurch bereitgestellte Ventil kann so ohne eine Veränderung der äußeren Form des äußeren Schlauchs geöffnet und wieder verschlossen werden. Hierdurch kann beispielsweise eine mechanische Reizung eines angrenzenden entzündeten Gewebes verhindert oder zumindest reduziert werden. Gleichzeitig kann das Schlauchsystem problemlos gekürzt werden und so dessen Länge an die jeweilige Situation angepasst werden. Hierzu muss lediglich das distale (erste) Ende der beiden Schläuche mit einem vorhandenen oder neuen Verbindungselement oder einer anderen Verbindung, wie einer durch Verschweißen erzeugten Verbindung, verschlossen werden. Die Vorrichtung und das Schlauchsystem sind kostengünstig aus Kunststoff herzustellen und können so als hygienisches Einmalprodukt zur Verfügung gestellt werden. Die Öffnungen im äußeren Schlauch werden dabei so verschlossen, dass im geschlossenen Zustand keine Hinterschneidungen im Zwischenraum zwischen dem äußeren Schlauch und dem inneren Schlauch entstehen, in die Gewebe einwachsen könnte und so ein Entfernen des Vorrichtung beziehungsweise des äußeren Schlauchs erschweren würde.

Die medizinische Vorrichtung besitzt eine außerhalb des Patienten zu betätigende Ventilfunktion. Die erfindungsgemäße Vorrichtung kann je nach anatomischer Situation des Implantationsortes oder je nach Tiefe des Hohlraums hinsichtlich ihrer Länge durch einfaches mechanisches Kürzen angepasst werden, ohne dass ein Funktionsverlust eintritt.

Der besondere Vorteil der erfindungsgemäßen Vorrichtung besteht darin, dass der medizinische Anwender jedes beliebige Fluid mit exakt definiertem Volumen applizieren kann. Bei wirkstoffhaltigen Fluiden können ein oder mehrere pharmazeutische Wirkstoffstoffe in dem Fluid in genau vorbestimmten Konzentrationen eingestellt werden. Dadurch ist es möglich, genau definierte Wirkstoffkonzentrationen in unmittelbarer Nähe der Öffnungen der Vorrichtung zu erreichen und damit zu behandeln. Ein weiterer Vorteil der Vorrichtung ist, dass die Öffnungen im äußeren Schlauch nur während der Applikation geöffnet sind und danach verschlossen werden, so dass kein Blut oder Gewebeflüssigkeit und auch kein sich bildendes Bindegewebe in den Zwischenraum zwischen den Schläuchen der Vorrichtung eindringen kann und dort Hinterschneidungen bilden kann, die beim Entfernen der Vorrichtung reißen und dadurch neue Irritationen des gerade behandelten Gewebes verursachen. Zudem werden Verstopfungen der Vorrichtung, insbesondere der Öffnungen im äußeren Schlauch, wirksam vermieden. Beim Schließen des Ventils werden durch die elastische Rückstellkraft des inneren Schlauchs Reste des Fluids aus dem Zwischenraum durch die Öffnungen in die Umgebung des äußeren Schlauchs ausgepresst. Es verbleiben keine Reste des Fluids in der Vorrichtung.

Eine beispielhafte erfindungsgemäße Vorrichtung zur lokalen Applikation von Fluiden mit Ventilfunktion ist zusammengesetzt aus
a) einem plastisch verformbaren äußeren Schlauch mit einem ersten und einem zweiten Schlauchendstück, wobei der äußere Schlauch mehrere Öffnungen in der Mantelfläche besitzt, welche den Innenraum des äußeren Schlauchs mit der Umgebung verbinden, wobei zumindest eine der Öffnungen in der Nähe des ersten Schlauchendstücks des äußeren Schlauchs angeordnet ist,
b) einem elastisch verformbaren inneren Schlauch, mit einem ersten und einem zweiten Schlauchendstück, mit einem Außendurchmesser gleich oder größer dem Innendurchmesser des äußeren Schlauchs, der koaxial im Innenraum des äußeren Schlauchs angeordnet ist, und der die Öffnungen des äußeren Schlauchs verschließt, wobei der äußere Schlauch den inneren Schlauch an dem ersten Schlauchendstück des inneren Schlauchs umschließt,
c) einem zweiten Fluid (die Flüssigkeit oder das Gas), das den Hohlraum im inneren Schlauch ausfüllt,
d) einer Vorrichtung zum Erzeugen von Unterdruck, die mit dem zweiten Schlauchendstück des inneren Schlauchs verbunden ist,
e) einem Konnektor, in dem der innere Schlauch aus dem zweiten Schlauchendstück des äußeren Schlauchs geführt wird, wobei der Konnektor mit dem Innenraum des äußeren Schlauchs flüssigkeits- und gasdurchlässig verbunden ist,
f) einem Verbindungselement als Verschluss, das das erste Schlauchendstück des äußeren Schlauchs und das erste Schlauchendstück des inneren Schlauchs flüssigkeits- und gasdicht verschließt und
g) wobei die Innenseite des äußeren Schlauchs mit der Außenfläche des inneren Schlauchs ein Ventil bildet, das durch Entfernen des zweiten Fluids aus dem Innenraum des inneren Schlauchs durch Einwirkung einer Unterdruck erzeugenden Vorrichtung unter Kontraktion des inneren Schlauchs senkrecht zur Längsachse des inneren Schlauchs geöffnet werden kann.

Das (erste) Fluid, das mit der Vorrichtung austragen wird, ist vorzugsweise ein pharmazeutisches Fluid. Unter dem Begriff "pharmazeutisches Fluid" werden wässrige und auch nichtwässrige Lösungen und Suspensionen von pharmazeutischen Wirkstoffen verstanden. Weiterhin werden unter dem Begriff "pharmazeutisches Fluid" auch Gemische und Lösungen von Gasen in Wasser, Wasser enthaltenden Flüssigkeiten und nichtwässrigen Flüssigkeiten verstanden. Der Begriff "pharmazeutisches Fluid" umfasst also auch Gase und Gasgemische.

Das Verbindungselement ist beispielsweise zusammengesetzt aus einem ersten rotationssymmetrischer Körper mit Außengewinde, wobei das Außengewinde einen größeren Außendurchmesser besitzt als der Innendurchmesser des inneren Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

In einer weiteren alternativen Ausgestaltungsform ist ein beispielhaftes Verbindungselement zusammengesetzt aus einem ersten rotationssymmetrischer Körper mit am Umfang umlaufenden Stegen, wobei die Stege einen größeren Außendurchmesser besitzen als der Innendurchmesser des inneren Schlauchs, und aus einem zweiten rotationssymmetrischen Körper mit einem Außendurchmesser kleiner oder gleich dem Außendurchmesser des äußeren Schlauchs, wobei die axiale Erstreckung des zweiten rotationssymmetrischen Körpers mindestens 5 mm ist, und wobei der erste rotationssymmetrische Körper axial mit dem zweiten rotationssymmetrischen Körper verbunden ist.

Ein beispielhaftes Verfahren zum Kürzen und Verschließen der Vorrichtung kann durch folgende nacheinander ablaufende Schritte charakterisiert sein:
a) Abschneiden des inneren Schlauchs und des äußeren Schlauchs ausgehend von den ersten (distalen) Schlauchendstücken, und
b) Einschrauben des Verbindungselements als Verschluss in das neu entstandene erste Schlauchendstück des inneren Schlauchs, wobei der Innenraum des äußeren Schlauchs flüssigkeits- und gasdicht verschlossen wird, wobei gleichzeitig der sich ausdehnende innere Schlauch am ersten Schlauchendstück flächig gegen die Innenseite des äußeren Schlauchs am ersten Schlauchendstück gepresst wird, und der Zwischenraum zwischen der Außenseite des äußeren Schlauchs und der Innenseite des inneren Schlauchs am ersten Schlauchende flüssigkeits- und gasdicht verschlossen wird.

Weiterhin ist ein beispielhaftes Verfahren zum Öffnen des Ventils einer erfindungsgemäßen Vorrichtung zur Applikation von Fluiden mit der Vorrichtung und nachfolgenden Verschluss des Ventils, das durch folgende nacheinander ablaufende Schritte charakterisiert ist:
a) Betätigung einer Einrichtung zum Erzeugen eines Unterdrucks im inneren Schlauch,
b) Heraussaugen der Flüssigkeit oder des Gases im inneren Schlauch mit der Einrichtung zum Erzeugen des Unterdrucks,
c) Zusammenziehen des inneren Schlauchs senkrecht zur Schlauchlängsachse,
d) Freilegen eines Zwischenraums zwischen der Außenseite des inneren Schlauchs und der Innenseite des äußeren Schlauchs, wobei die Öffnungen des äußeren Schlauchs flüssigkeits- und gasdurchlässig mit dem Zwischenraum verbunden wird,
e) Einpressen eines Fluids durch den Konnektor in das zweite Schlauchendstück in den Zwischenraum zwischen der Außenseite des inneren Schlauchs und der Innenseite des äußeren Schlauchs,
f) Austreten des Fluids aus den Öffnungen im äußeren Schlauch in die Umgebung,
g) Abbau des Unterdrucks im inneren Schlauch durch Zurückstellung der Einrichtung zur Erzeugung von Unterdruck,
h) Rückfließen der Flüssigkeit oder des Gases aus der Einrichtung zur Erzeugung von Unterdruck in den inneren Schlauch, und
i) elastische Rückstellung des inneren Schlauchs unter Anpressung der Außenseite des inneren Schlauchs an die Innenseite des äußeren Schlauchs unter Verschluss der Öffnungen des äußeren Schlauchs.

Im Folgenden werden weitere Ausführungsbeispiele der Erfindung anhand von vierzehn schematisch dargestellten Figuren erläutert, ohne jedoch dabei die Erfindung zu beschränken. Dabei zeigt:
Figur 1: eine schematische Ansicht einer beispielhaften ersten erfindungsgemäßen medizinischen Vorrichtung zur lokalen Applikation eines Fluids;
Figur 2: eine schematische Ansicht einer beispielhaften alternativen zweiten erfindungsgemäßen medizinischen Vorrichtung zur lokalen Applikation eines Fluids;
Figur 3: eine schematische Ansicht einer beispielhaften alternativen dritten erfindungsgemäßen medizinischen Vorrichtung zur lokalen Applikation eines Fluids;
Figur 4: eine schematische Ansicht einer beispielhaften alternativen vierten erfindungsgemäßen medizinischen Vorrichtung zur lokalen Applikation eines Fluids;
Figur 5: zwei schematische perspektivische Außenansichten der ersten Vorrichtung nach Figur 1;
Figur 6: eine schematische Längsquerschnittansicht der ersten Vorrichtung nach den Figuren 1 und 5 in einem Ausgangszustand;
Figur 7: eine schematische Längsquerschnittansicht der ersten Vorrichtung nach den Figuren 1, 5 und 6 im Ausgangszustand, wobei der Längsquerschnitt senkrecht zu dem nach Figur 6 verläuft;
Figur 8: zwei schematische perspektivische und senkrecht zueinander geschnittene Längsquerschnittansichten der ersten Vorrichtung nach den Figuren 1 und 5 bis 7 mit einer angeschlossenen Spritze zum Injizieren eines Fluids;
Figur 9: eine Ausschnittvergrößerung der Längsquerschnittansichten nach Figur 8 mit entarretiertem Druckkolben;
Figur 10: eine Ausschnittvergrößerung zweier schematischer perspektivischer und senkrecht zueinander geschnittener Längsquerschnittansichten der ersten Vorrichtung nach den Figuren 1 und 5 bis 9, wobei die Öffnungen im äußeren Schlauch durch Zurückziehen des Druckkolbens geöffnet sind;
Figur 11: zwei schematische perspektivische und senkrecht zueinander geschnittene Längsquerschnittansichten der ersten Vorrichtung nach den Figuren 1 und 5 bis 10 während der Applikation des Fluids durch die geöffneten Öffnungen;
Figur 12: eine Ausschnittvergrößerung der Längsquerschnittansichten nach Figur 11;
Figur 13: zwei schematische perspektivische und senkrecht zueinander geschnittene Längsquerschnittansichten der ersten Vorrichtung nach den Figuren 1 und 5 bis 12, wobei die Öffnungen im äußeren Schlauch durch Eindrücken des Druckkolbens wieder geschlossen sind; und
Figur 14: eine Ausschnittvergrößerung der Längsquerschnittansichten nach Figur 13.

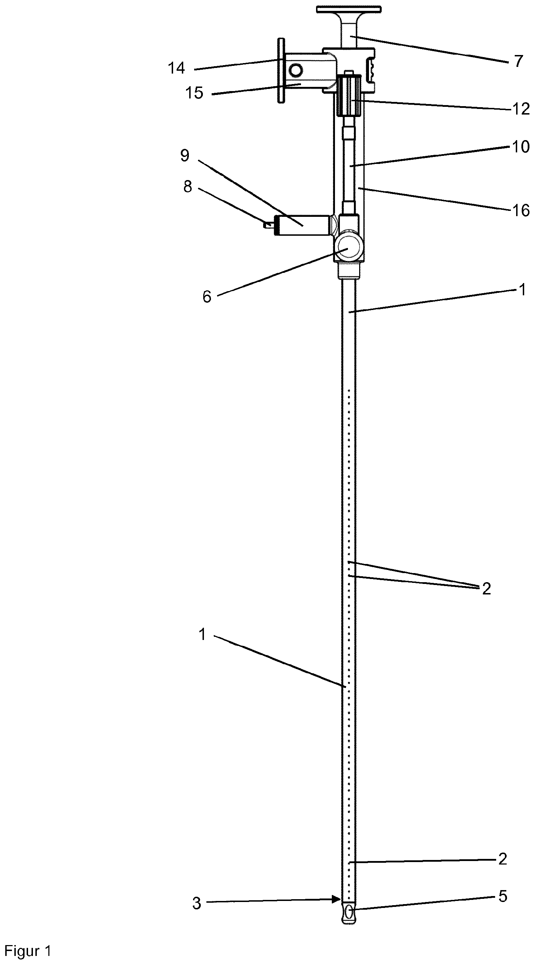

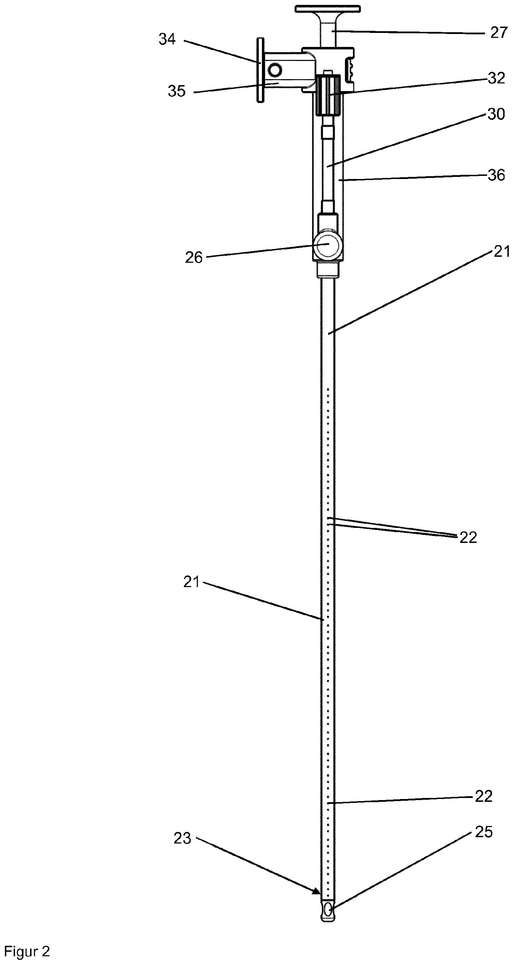

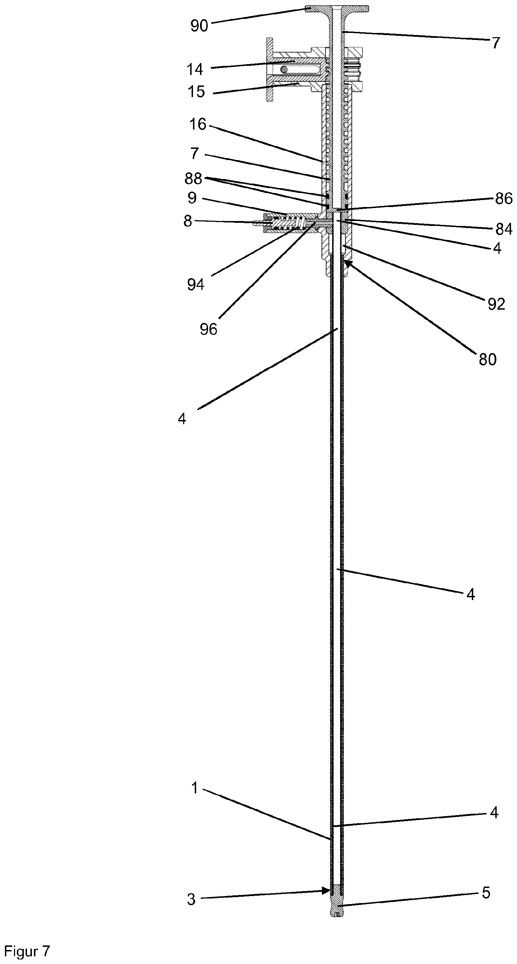

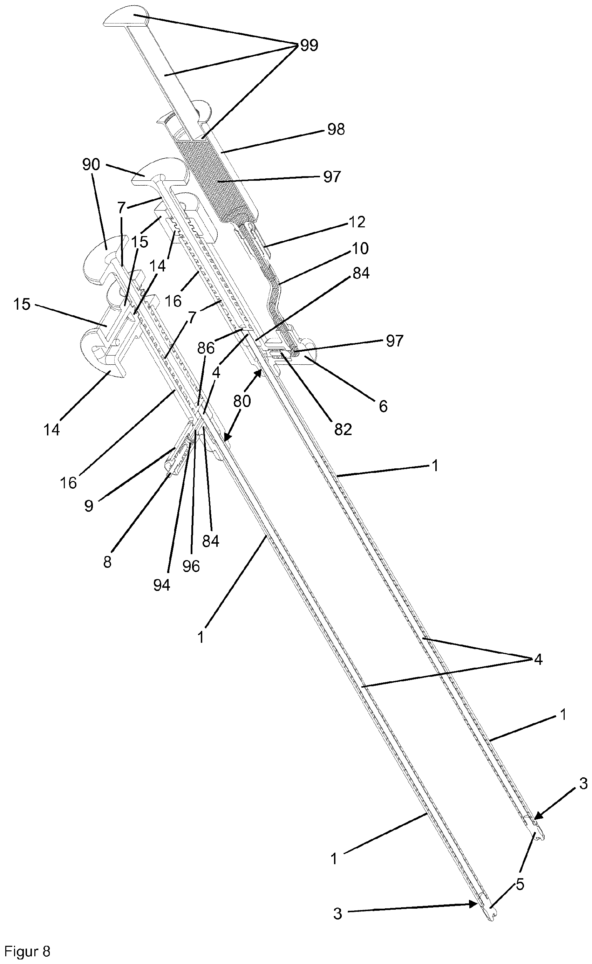

Die Figuren 1 und 5 bis 14 zeigen eine erste beispielhafte erfindungsgemäße Vorrichtung in unterschiedlichen Darstellungen. Die Figuren 2, 3 und 4 zeigen drei andere Ausführungsbeispiele der vorliegenden Erfindung. In den Figuren 8 bis 14 ist zum ersten Ausführungsbeispiel der Ablauf eines erfindungsgemäßen Verfahrens gezeigt, der sich ohne weiteres auf die anderen Ausführungsbeispiele nach den Figuren 2 bis 4 übertragen lässt.

Die erste beispielhafte erfindungsgemäße Vorrichtung, die in den Figuren 1 und 5 bis 14 gezeigt ist, hat an der vorderen distalen Seite einen äußeren Schlauch 1, in dem eine Vielzahl von durch die Schlauchwandung durchgehenden Öffnungen 2 angeordnet sind. Der äußere Schlauch 1 besteht aus einem plastisch verformbaren Material, so dass seine Form an die Form eines Hohlraums angepasst werden kann.

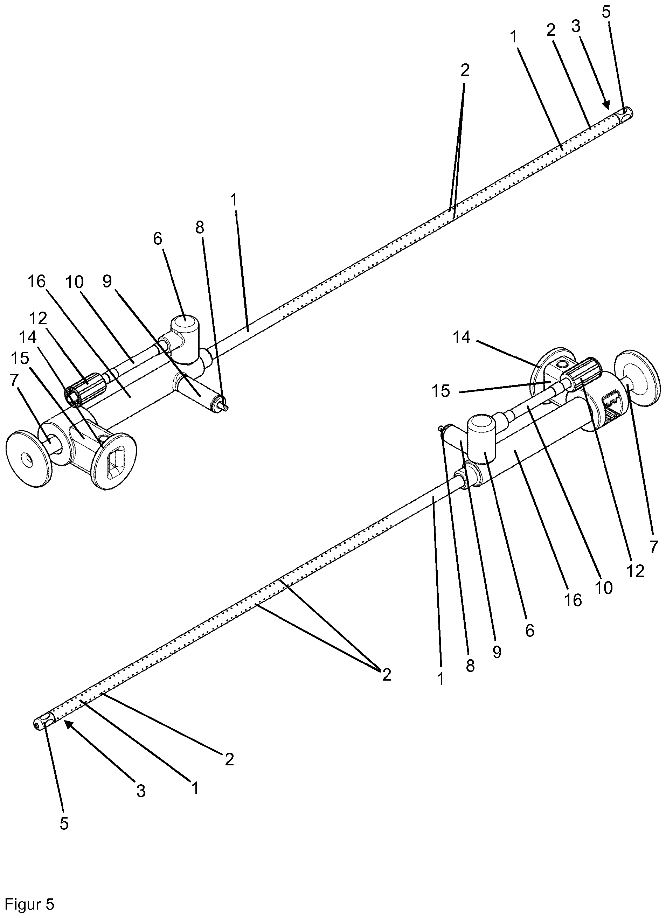

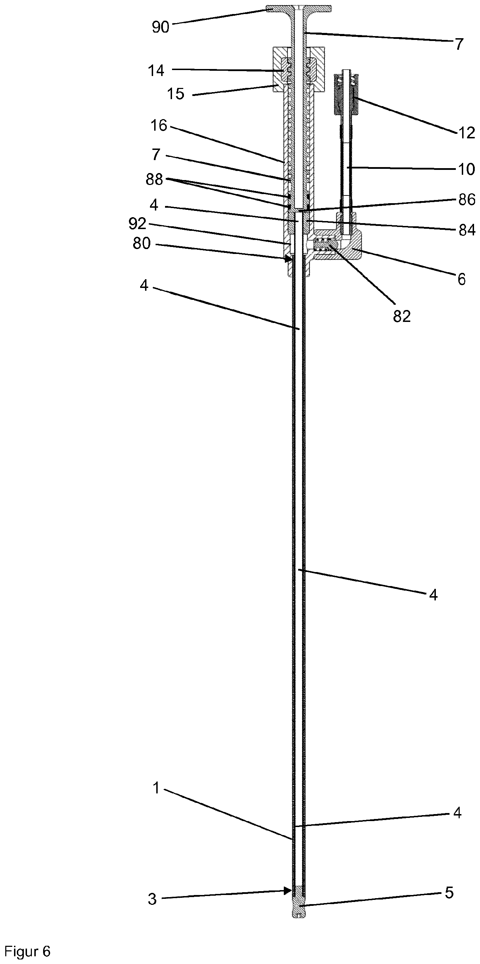

Der äußere Schlauch 1 ist an seinem distalen ersten Ende 3 mit einem inneren Schlauch 4 verbunden, der koaxial innerhalb des äußeren Schlauchs 1 angeordnet ist. Der innere Schlauch 4 ist in den Figuren 1 und 5 nicht erkennbar aber in den Querschnittansichten der Figuren 6 bis 14 zu sehen. Der innere Schlauch 4 hat einen Außendurchmesser, der im entspannten Zustand (siehe Figuren 6 bis 9 und 13 und 14) mindestens genausgroß ist, wie der Innendurchmesser des äußeren Schlauchs 1. Dadurch verschließt der innere Schlauch 4 im entspannten Zustand die Öffnungen 2 im äußeren Schlauch 1 auf der Innenseite des äußeren Schlauchs 1. Zudem verbleibt zwischen dem äußeren Schlauch 1 und dem inneren Schlauch 4 kein Volumen im Zwischenraum. Der innere Schlauch 4 bildet zusammen mit dem äußeren Schlauch 1 ein Ventil zum Öffnen und Schließen der Öffnungen 2 im äußeren Schlauch 1.

Der innere Schlauch 4 ist mit dem äußeren Schlauch 1 über ein Verbindungselement 5 verbunden. Hierzu hat das Verbindungselement 5 einen zylindrischen Vorsprung mit einem Außengewinde, das in das offene distale Ende des inneren Schlauchs 4 eingeschraubt ist. Das Außengewinde des zylindrischen Vorsprungs schneidet sich beim Einschrauben des Verbindungselements 5 ein Innengewinde in die Innenwand des inneren Schlauchs 4. Dabei dehnt sich der innere Schlauch 4 und wird an den äußeren Schlauch 1 gepresst, so dass der Zwischenraum zwischen dem äußeren Schlauch 1 und dem inneren Schlauch 4 gasdicht und flüssigkeitsdicht verschlossen ist. Zudem verschließt das Verbindungselement 5 den inneren Schlauch 4 an seinem distalen Ende gasdicht und flüssigkeitsdicht.

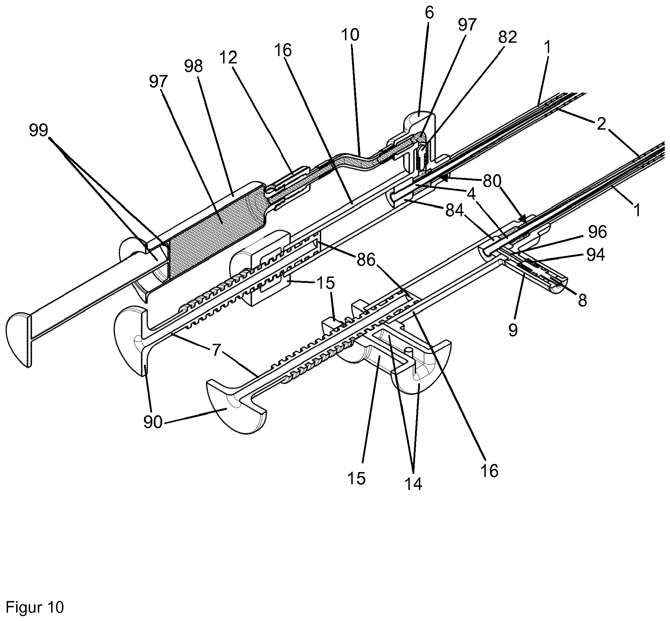

An einem proximalen zweiten Ende 80 (siehe Figuren 6 bis 14) ist der äußere Schlauch 1 mit einem Gehäuse 16 verbunden. Der Zwischenraum zwischen dem äußeren Schlauch 1 und dem inneren Schlauch 4 ist mit einem Konnektor 6 verbunden, durch den ein Fluid 97 (siehe Figuren 8 bis 14) in diesen Zwischenraum eingeleitet werden kann. Das Gehäuse 16 bildet in seinem Inneren einen Hubraum für einen Druckkolben 7, der von außerhalb der Vorrichtung bedienbar ist. Der Hubraum für den Druckkolben 7 ist mit dem Innenraum des inneren Schlauchs 4 verbunden. Im Inneren des Hubraums und des inneren Schlauchs 4 befindet sich eine Flüssigkeit oder ein Gas. Durch ein Vergrößern des Hubraums durch Herausziehen des Druckkolbens 7 aus dem Gehäuse 16 wird Flüssigkeit oder Gas zumindest teilweise aus dem inneren Schlauch 4 abgesaugt, so dass die Schlauchwandungen des inneren Schlauchs 4 kontrahieren und der innere Schlauch 4 in einen radial kontrahierten Zustand überführbar ist (siehe die Figuren 10 bis 12). Das Gehäuse 16 mit dem Hubraum bildet zusammen mit dem Druckkolben 7 eine Kolbenspritze, die als Bedienelement zum Verändern des Gasdrucks oder des Volumens der Flüssigkeit in dem inneren Schlauch 4 bezeichnet werden kann.

An dem Gehäuse 16 ist ferner ein Indikator befestigt, mit dem von außen optisch abgelesen werden kann, ob sich der innere Schlauch 4 im radial kontrahierten Zustand oder im expandierten Zustand befindet. Hierzu ist ein Stift 8 in Signalfarbe axial beschränkt beweglich in einem Gehäuse 9 für den Stift 8 gelagert. Die Farbe des Gehäuses 9 hat vorzugsweise einen hohen Kontrast zu der Farbe des Stifts 8. Wenn der Stift 8 aus dem Gehäuse 9 für den Stift 8 herausragt, befindet sich der innere Schlauch 4 im expandierten Zustand. Wenn der Stift 8 in dem Gehäuse 9 für den Stift 8 versenkt ist, befindet sich der innere Schlauch 4 im radial kontrahierten Zustand.

An dem Konnektor 6 ist eine Leitung 10 angeschlossen, die den Konnektor 6 mit einem Anschlussadapter 12, wie beispielsweise einem Luer-Lock-Adapter, verbindet.

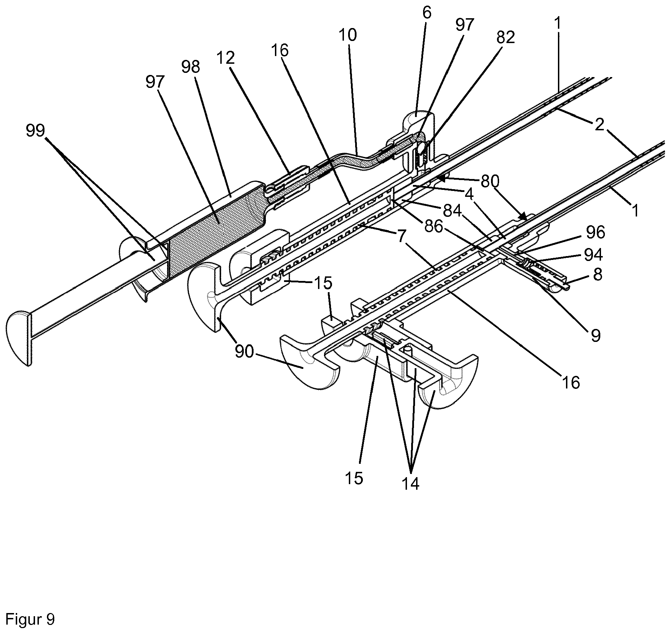

An dem Gehäuse 16 ist des Weiteren eine Arretierung 14 zum Arretieren des Druckkolbens 7 angeordnet. Hierzu ist die Arretierung 14 in einem Gehäuse 15 für die Arretierung 14 senkrecht zur Bewegungsrichtung des Druckkolbens 7 gelagert. Der Druckkolben 7 weist an seiner Außenseite eine Vielzahl von Nuten am äußeren Umfang auf, in die dazu passende Leisten der Arretierung 14 eingreifen, wenn der Druckkolben 7 arretiert ist (siehe Figuren 6 bis 8 und 10 bis 14). Durch Herausziehen der Arretierung 14 aus dem Gehäuse 15 für die Arretierung 14 (siehe Figur 9) wird der Druckkolben 7 entarretiert und kann axial in dem Hubraum des Gehäuses 16 bewegt werden. Es kann vorgesehen sein, dass die Arretierung 14 mit einer Druckfeder (nicht gezeigt) so in dem Gehäuse 15 für die Arretierung 14 gelagert ist, dass die Arretierung 14 auf den Druckkolben 7 geschoben wird und diesen arretiert.

Die zweite beispielhafte erfindungsgemäße Vorrichtung, die in Figur 2 gezeigt ist, gleicht bis auf einen fehlenden Druckindikator dem ersten Ausführungsbeispiel nach den Figuren 1 und 5 bis 14. Die zweite beispielhafte erfindungsgemäße Vorrichtung hat an der vorderen distalen Seite einen äußeren Schlauch 21, in dem eine Vielzahl von durch die Schlauchwandung durchgehenden Öffnungen 22 angeordnet sind. Der äußere Schlauch 21 besteht aus einem plastisch verformbaren Material, so dass seine Form an die Form eines Hohlraums angepasst werden kann.

Der äußere Schlauch 21 ist an seinem distalen ersten Ende 23 mit einem inneren Schlauch (in Figur 2 nicht zu sehen) verbunden, der koaxial innerhalb des äußeren Schlauchs 21 angeordnet ist oder der entlang einer Verbindungslinie mit der Innenwand des äußeren Schlauchs 21 verbunden ist, vorzugsweise ohne dabei entlang der Verbindungslinie die Öffnungen 22 abzudecken. Der innere Schlauch hat einen Außendurchmesser, der im entspannten Zustand mindestens genausgroß ist, wie der Innendurchmesser des äußeren Schlauchs 21. Dadurch verschließt der innere Schlauch im entspannten Zustand die Öffnungen 22 im äußeren Schlauch 21 auf der Innenseite des äußeren Schlauchs 21. Zudem verbleibt zwischen dem äußeren Schlauch 21 und dem inneren Schlauch kein Volumen im Zwischenraum. Der innere Schlauch bildet zusammen mit dem äußeren Schlauch 21 ein Ventil zum Öffnen und Schließen der Öffnungen 22 im äußeren Schlauch 21.

Der innere Schlauch ist mit dem äußeren Schlauch 21 über ein Verbindungselement 25 verbunden. Hierzu hat das Verbindungselement 25 einen zylindrischen Vorsprung mit einem Außengewinde, das in das offene distale Ende des inneren Schlauchs eingeschraubt ist, oder einen zylindrischen oder konischen Vorsprung mit Leisten, die sich in das Material des inneren Schlauchs drücken oder schneiden. Dabei dehnt sich der innere Schlauch und wird an den äußeren Schlauch 21 gepresst, so dass der Zwischenraum zwischen dem äußeren Schlauch 21 und dem inneren Schlauch gasdicht und flüssigkeitsdicht verschlossen ist. Zudem verschließt das Verbindungselement 25 den inneren Schlauch an seinem distalen Ende gasdicht und flüssigkeitsdicht.

An einem proximalen zweiten Ende des äußeren Schlauchs 21, das im Bereich eines Konnektors 26 im inneren eines Gehäuses 36 angeordnet ist und in Figur 2 nicht zu sehen ist, ist der äußere Schlauch 21 mit dem Gehäuse 36 verbunden. Der Zwischenraum zwischen dem äußeren Schlauch 21 und dem inneren Schlauch ist mit dem Konnektor 26 für ein Fluid (in Figur 2 nicht gezeigt) durchgehend verbunden. Durch den Konnektor 26 kann so das Fluid in diesen Zwischenraum eingeleitet werden kann. Das Gehäuse 36 bildet in seinem Inneren einen Hubraum für einen Druckkolben 27, der von außerhalb der Vorrichtung bedienbar ist. Der Hubraum für den Druckkolben 27 ist mit dem Innenraum des inneren Schlauchs verbunden. Im Inneren des Hubraums und des inneren Schlauchs befindet sich eine Flüssigkeit oder ein Gas. Durch ein Vergrößern des Hubraums durch Herausziehen des Druckkolbens 27 aus dem Gehäuse 36 wird Flüssigkeit oder Gas aus dem inneren Schlauch zumindest teilweise abgesaugt, so dass die Schlauchwandungen des inneren Schlauchs kontrahieren und der innere Schlauch in einen radial kontrahierten Zustand überführbar ist. Dadurch sind die Öffnungen 22 im äußeren Schlauch 21 zu öffnen und das durch den inneren Schlauch und den äußeren Schlauch 21 mit den Öffnungen 22 gebildete Ventil wird auf diese Weise bedient beziehungsweise geöffnet. Das Gehäuse 36 mit dem Hubraum bildet zusammen mit dem Druckkolben 27 eine Kolbenspritze, die als Bedienelement zum Verändern des Gasdrucks oder des Volumens der Flüssigkeit in dem inneren Schlauch bezeichnet werden kann.

An dem Konnektor 26 ist eine Leitung 30 angeschlossen, die den Konnektor 26 mit einem Anschlussadapter 32, wie beispielsweise einem Luer-Lock-Adapter, verbindet.

An dem Gehäuse 36 ist des Weiteren eine Arretierung 34 zum Arretieren des Druckkolbens 27 angeordnet. Hierzu ist die Arretierung 34 in einem Gehäuse 35 für die Arretierung 34 senkrecht zur Bewegungsrichtung des Druckkolbens 27 gelagert. Der Druckkolben 27 weist an seiner Außenseite eine Vielzahl von Nuten am äußeren Umfang auf, in die dazu passende Leisten der Arretierung 34 eingreifen, wenn der Druckkolben 27 arretiert ist. Durch Herausziehen der Arretierung 34 aus dem Gehäuse 35 für die Arretierung 34 wird der Druckkolben 27 entarretiert und kann axial in dem Hubraum des Gehäuses 36 bewegt werden.

Es kann vorgesehen sein, dass die Arretierung 34 mit einer Druckfeder (nicht gezeigt) so in dem Gehäuse 35 für die Arretierung 34 gelagert ist, dass die Arretierung 34 auf den Druckkolben 27 geschoben wird und diesen arretiert.

Das zweite Ausführungsbeispiel nach Figur 2 unterscheidet sich von dem ersten Ausführungsbeispiel nach den Figuren 1 und 5 bis 14 dadurch, dass kein zusätzlicher Indikator vorgesehen ist, an dem abgelesen werden könnte, ob sich der innere Schlauch im radial kontrahierten Zustand befindet. Lediglich durch die Stellung des Druckkolbens 27 kann der Anwender Rückschlüsse darauf ziehen, ob sich der innere Schlauch im radial kontrahierten Zustand befindet und damit die Öffnungen 22 geöffnet sind, ohne dass eine zusätzliche Messung dies sicherstellen würde. Die Vorrichtung gemäß dem zweiten Ausführungsbeispiel ist dadurch kostengünstiger aber auch weniger sicher. Für einfache Spülvorgänge, bei denen das Schlauchsystem nicht bei einem Patienten implantiert werden muss oder der Rest der Vorrichtung bei implantiertem Schlauchsystem leicht gewechselt werden kann, ist dies ausreichend.

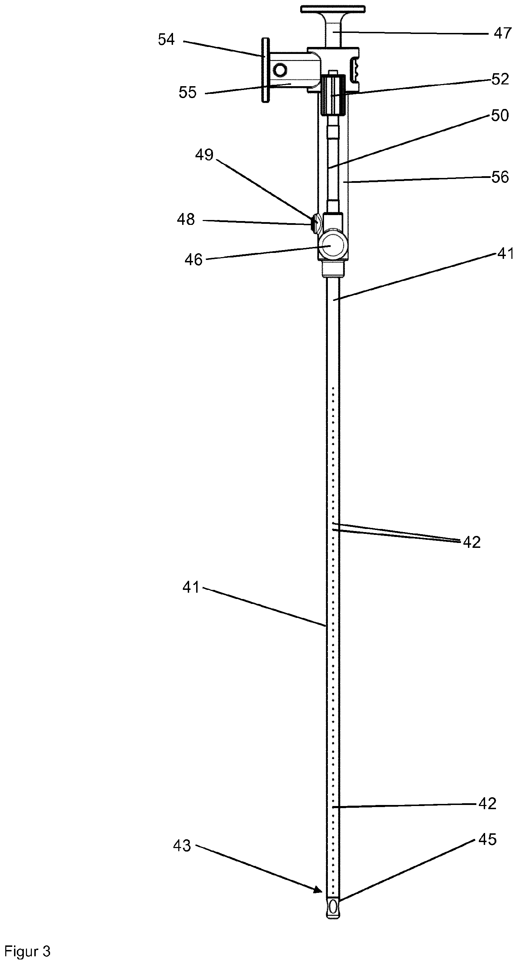

Die dritte beispielhafte erfindungsgemäße Vorrichtung, die in Figur 3 gezeigt ist, gleicht bis auf einen sich unterscheidenden Druckindikator dem ersten Ausführungsbeispiel nach den Figuren 1 und 5 bis 14. Die dritte beispielhafte erfindungsgemäße Vorrichtung hat an der vorderen distalen Seite einen äußeren Schlauch 41, in dem eine Vielzahl von durch die Schlauchwandung durchgehenden Öffnungen 42 angeordnet sind. Der äußere Schlauch 41 besteht aus einem plastisch oder elastisch verformbaren Material, so dass seine Form an die Form eines Hohlraums angepasst werden kann oder sich selbst anpasst.

Der äußere Schlauch 41 ist an seinem distalen ersten Ende 43 mit einem inneren Schlauch (in Figur 3 nicht zu sehen) verbunden, der koaxial innerhalb des äußeren Schlauchs 41 angeordnet ist oder der entlang einer Verbindungslinie mit der Innenwand des äußeren Schlauchs 41 verbunden ist, vorzugsweise ohne dabei entlang der Verbindungslinie die Öffnungen 42 abzudecken. Der innere Schlauch hat einen Außendurchmesser, der im entspannten Zustand mindestens genausgroß ist, wie der Innendurchmesser des äußeren Schlauchs 41. Dadurch verschließt der innere Schlauch im entspannten Zustand die Öffnungen 42 im äußeren Schlauch 41 auf der Innenseite des äußeren Schlauchs 41. Zudem verbleibt zwischen dem äußeren Schlauch 41 und dem inneren Schlauch kein Volumen im Zwischenraum. Der innere Schlauch bildet zusammen mit dem äußeren Schlauch 41 ein Ventil zum Öffnen und Schließen der Öffnungen 42 im äußeren Schlauch 41.

Der innere Schlauch ist mit dem äußeren Schlauch 41 über ein Verbindungselement 45 verbunden. Hierzu hat das Verbindungselement 45 einen zylindrischen Vorsprung mit einem Außengewinde, das in das offene distale Ende des inneren Schlauchs eingeschraubt ist, oder einen zylindrischen oder konischen Vorsprung mit Leisten, die sich in das Material des inneren Schlauchs drücken oder schneiden. Dabei dehnt sich der innere Schlauch und wird an den äußeren Schlauch 41 gepresst, so dass der Zwischenraum zwischen dem äußeren Schlauch 41 und dem inneren Schlauch gasdicht und flüssigkeitsdicht verschlossen ist. Zudem verschließt das Verbindungselement 45 den inneren Schlauch an seinem distalen Ende gasdicht und flüssigkeitsdicht.

An einem proximalen zweiten Ende des äußeren Schlauchs 41, das im Bereich eines Konnektors 46 im inneren eines Gehäuses 56 angeordnet ist und in Figur 3 nicht zu sehen ist, ist der äußere Schlauch 41 mit dem Gehäuse 56 verbunden. Der Zwischenraum zwischen dem äußeren Schlauch 41 und dem inneren Schlauch ist mit dem Konnektor 56 für ein Fluid (in Figur 3 nicht gezeigt) durchgehend verbunden. Durch den Konnektor 46 kann so das Fluid in diesen Zwischenraum eingeleitet werden kann. Das Gehäuse 56 bildet in seinem Inneren einen Hubraum für einen Druckkolben 47, der von außerhalb der Vorrichtung bedienbar ist. Der Hubraum für den Druckkolben 47 ist mit dem Innenraum des inneren Schlauchs verbunden. Im Inneren des Hubraums und des inneren Schlauchs befindet sich eine Flüssigkeit oder ein Gas. Durch ein Vergrößern des Hubraums durch Herausziehen des Druckkolbens 47 aus dem Gehäuse 56 wird Flüssigkeit oder Gas aus dem inneren Schlauch zumindest teilweise abgesaugt, so dass die Schlauchwandungen des inneren Schlauchs kontrahieren und der innere Schlauch in einen radial kontrahierten Zustand überführbar ist. Dadurch sind die Öffnungen 42 im äußeren Schlauch 41 zu öffnen und das durch den inneren Schlauch und den äußeren Schlauch 41 mit den Öffnungen 42 gebildete Ventil wird auf diese Weise bedient beziehungsweise geöffnet. Das Gehäuse 56 mit dem Hubraum bildet zusammen mit dem Druckkolben 47 eine Kolbenspritze, die als Bedienelement zum Verändern des Gasdrucks oder des Volumens der Flüssigkeit in dem inneren Schlauch bezeichnet werden kann.

An dem Gehäuse 56 ist ferner ein Indikator befestigt, mit dem von außen optisch abgelesen werden kann, ob sich der innere Schlauch im radial kontrahierten Zustand oder im expandierten Zustand befindet. Hierzu ist eine dehnbare Membran 48 in Signalfarbe axial an einem Anschluss 49 für die Membran 48 befestigt. Wenn die Membran 48 aus dem Anschluss 49 für die Membran 48 konvex hervorsteht, befindet sich der innere Schlauch im expandierten Zustand. Wenn die Membran 48 sich in den Anschluss 49 für die Membran 48 konkav hineinzieht, befindet sich der innere Schlauch im radial kontrahierten Zustand.

An dem Konnektor 46 ist eine Leitung 50 angeschlossen, die den Konnektor 46 mit einem Anschlussadapter 52, wie beispielsweise einem Luer-Lock-Adapter, verbindet.

An dem Gehäuse 56 ist des Weiteren eine Arretierung 54 zum Arretieren des Druckkolbens 47 angeordnet. Hierzu ist die Arretierung 54 in einem Gehäuse 55 für die Arretierung 54 senkrecht zur Bewegungsrichtung des Druckkolbens 47 gelagert. Der Druckkolben 47 weist an seiner Außenseite eine Vielzahl von Nuten am äußeren Umfang auf, in die dazu passende Leisten der Arretierung 54 eingreifen, wenn der Druckkolben 47 arretiert ist. Durch Herausziehen der Arretierung 54 aus dem Gehäuse 55 für die Arretierung 54 wird der Druckkolben 47 entarretiert und kann axial in dem Hubraum des Gehäuses 56 bewegt werden. Es kann vorgesehen sein, dass die Arretierung 54 mit einer Druckfeder (nicht gezeigt) so in dem Gehäuse 55 für die Arretierung 54 gelagert ist, dass die Arretierung 54 auf den Druckkolben 47 geschoben wird und diesen arretiert.

Das dritte Ausführungsbeispiel nach Figur 3 unterscheidet sich von dem ersten Ausführungsbeispiel nach den Figuren 1 und 5 bis 14 dadurch, dass ein anderer Indikator vorgesehen ist, an dem abgelesen werden kann, ob sich der innere Schlauch im radial kontrahierten Zustand befindet.

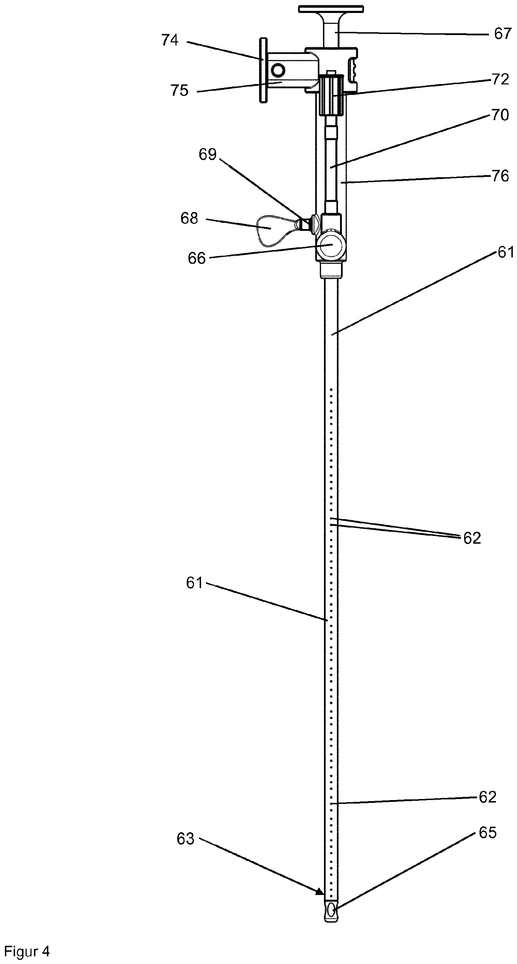

Die vierte beispielhafte erfindungsgemäße Vorrichtung, die in Figur 4 gezeigt ist, gleicht bis auf einen sich unterscheidenden Druckindikator dem ersten Ausführungsbeispiel nach den Figuren 1 und 5 bis 14. Die dritte beispielhafte erfindungsgemäße Vorrichtung hat an der vorderen distalen Seite einen äußeren Schlauch 61, in dem eine Vielzahl von durch die Schlauchwandung durchgehenden Öffnungen 62 angeordnet sind. Der äußere Schlauch 61 besteht aus einem plastisch oder elastisch verformbaren Material, so dass seine Form an die Form eines Hohlraums angepasst werden kann oder sich selbst anpasst.

Der äußere Schlauch 61 ist an seinem distalen ersten Ende 63 mit einem inneren Schlauch (in Figur 4 nicht zu sehen) verbunden, der koaxial innerhalb des äußeren Schlauchs 61 angeordnet ist. Der innere Schlauch hat einen Außendurchmesser, der im entspannten Zustand mindestens genausgroß ist, wie der Innendurchmesser des äußeren Schlauchs 61. Dadurch verschließt der innere Schlauch im entspannten Zustand die Öffnungen 62 im äußeren Schlauch 61 auf der Innenseite des äußeren Schlauchs 61. Zudem verbleibt zwischen dem äußeren Schlauch 61 und dem inneren Schlauch kein Volumen im Zwischenraum. Der innere Schlauch bildet zusammen mit dem äußeren Schlauch 61 ein Ventil zum Öffnen und Schließen der Öffnungen 62 im äußeren Schlauch 61.

Der innere Schlauch ist mit dem äußeren Schlauch 61 über ein Verbindungselement 65 verbunden. Hierzu hat das Verbindungselement 65 einen zylindrischen Vorsprung mit einem Außengewinde, das in das offene distale Ende des inneren Schlauchs eingeschraubt ist, oder einen zylindrischen oder konischen Vorsprung mit Leisten, die sich in das Material des inneren Schlauchs drücken oder schneiden. Dabei dehnt sich der innere Schlauch und wird an den äußeren Schlauch 61 gepresst, so dass der Zwischenraum zwischen dem äußeren Schlauch 61 und dem inneren Schlauch gasdicht und flüssigkeitsdicht verschlossen ist. Zudem verschließt das Verbindungselement 65 den inneren Schlauch an seinem distalen Ende gasdicht und flüssigkeitsdicht.

An einem proximalen zweiten Ende des äußeren Schlauchs 61, das im Bereich eines Konnektors 66 im inneren eines Gehäuses 76 angeordnet ist und in Figur 4 nicht zu sehen ist, ist der äußere Schlauch 61 mit dem Gehäuse 76 verbunden. Der Zwischenraum zwischen dem äußeren Schlauch 61 und dem inneren Schlauch ist mit dem Konnektor 76 für ein Fluid (in Figur 4 nicht gezeigt) durchgehend verbunden. Durch den Konnektor 66 kann so das Fluid in diesen Zwischenraum eingeleitet werden kann. Das Gehäuse 76 bildet in seinem Inneren einen Hubraum für einen Druckkolben 67, der von außerhalb der Vorrichtung bedienbar ist. Der Hubraum für den Druckkolben 67 ist mit dem Innenraum des inneren Schlauchs verbunden. Im Inneren des Hubraums und des inneren Schlauchs befindet sich eine Flüssigkeit oder ein Gas. Durch ein Vergrößern des Hubraums durch Herausziehen des Druckkolbens 67 aus dem Gehäuse 76 wird Flüssigkeit oder Gas aus dem inneren Schlauch zumindest teilweise abgesaugt, so dass die Schlauchwandungen des inneren Schlauchs kontrahieren und der innere Schlauch in einen radial kontrahierten Zustand überführbar ist. Dadurch sind die Öffnungen 62 im äußeren Schlauch 61 zu öffnen und das durch den inneren Schlauch und den äußeren Schlauch 61 mit den Öffnungen 62 gebildete Ventil wird auf diese Weise bedient beziehungsweise geöffnet. Das Gehäuse 76 mit dem Hubraum bildet zusammen mit dem Druckkolben 67 eine Kolbenspritze, die als Bedienelement zum Verändern des Gasdrucks oder des Volumens der Flüssigkeit in dem inneren Schlauch bezeichnet werden kann.

An dem Gehäuse 76 ist ferner ein Indikator befestigt, mit dem von außen optisch abgelesen werden kann, ob sich der innere Schlauch im radial kontrahierten Zustand oder im expandierten Zustand befindet. Hierzu ist ein dehnbarer Ballon 68 in Signalfarbe axial an einem Anschluss 69 für den Ballon 68 befestigt. Das Gas oder die Flüssigkeit im Inneren des inneren Schlauchs ist in dem Ballon 68 enthalten und mit dem Ballon 68 durchgehend verbunden. Wenn der Ballon 68 ausgedehnt beziehungsweise aufgeblasen ist, befindet sich der innere Schlauch im expandierten Zustand. Wenn der Ballon 68 kompakt oder schlaff ist, befindet sich der innere Schlauch im radial kontrahierten Zustand.

An dem Konnektor 66 ist eine Leitung 70 angeschlossen, die den Konnektor 66 mit einem Anschlussadapter 72, wie beispielsweise einem Luer-Lock-Adapter, verbindet.

An dem Gehäuse 76 ist des Weiteren eine Arretierung 74 zum Arretieren des Druckkolbens 67 angeordnet. Hierzu ist die Arretierung 74 in einem Gehäuse 75 für die Arretierung 74 senkrecht zur Bewegungsrichtung des Druckkolbens 67 gelagert. Der Druckkolben 67 weist an seiner Außenseite eine Vielzahl von Nuten am äußeren Umfang auf, in die dazu passende Leisten der Arretierung 74 eingreifen, wenn der Druckkolben 67 arretiert ist. Durch Herausziehen der Arretierung 74 aus dem Gehäuse 75 für die Arretierung 74 wird der Druckkolben 67 entarretiert und kann axial in dem Hubraum des Gehäuses 76 bewegt werden. Es kann vorgesehen sein, dass die Arretierung 74 mit einer Druckfeder (nicht gezeigt) so in dem Gehäuse 75 für die Arretierung 74 gelagert ist, dass die Arretierung 74 auf den Druckkolben 67 geschoben wird und diesen arretiert.

Das vierte Ausführungsbeispiel nach Figur 4 unterscheidet sich von dem ersten Ausführungsbeispiel nach den Figuren 1 und 5 bis 14 dadurch, dass ein anderer Indikator vorgesehen ist, an dem abgelesen werden kann, ob sich der innere Schlauch im radial kontrahierten Zustand befindet.

In den Figuren 6 bis 14 ist der innere Aufbau und die Arbeitsweise der Vorrichtung nach dem ersten erfindungsgemäßen Ausführungsbeispiel zu erkennen. Die Arbeitsweise und der innere Aufbau sind sehr weitegehend beziehungsweise bis auf die Funktionsweise des jeweiligen Indikators vollständig auf die anderen Ausführungsbeispiele nach den Figuren 2 bis 4 übertragbar. Im Folgenden wird dabei der Ablauf eines erfindungsgemäßen Verfahrens beispielhaft anhand der ersten erfindungsgemäßen Vorrichtung beschrieben.

Das proximale zweite Ende 80 des äußeren Schlauchs 1 ist an seiner Außenseite flüssigkeitsdicht mit dem Gehäuse 16 verbunden. Im Inneren des Konnektors 6 ist ein Rückschlagventil 82 angeordnet, das mit einer Feder gegen einen Ventilsitz im Inneren des Konnektors 6 gedrückt wird. Mit dem Rückschlagventil 82 wird ein Rückfluss des Fluids 97 aus dem Gehäuse 16 in die Leitung 10 verhindert. Das proximale Ende des inneren Schlauchs 4 ist mit einer Hülse 84 in dem Gehäuse 16 fixiert und dort auf diese Weise gasdicht und flüssigkeitsdicht verschlossen. Das proximale Ende des inneren Schlauchs 4 ist aus dem proximalen zweiten Ende 80 des äußeren Schlauchs 1 geführt und steht aus dem äußeren Schlauch 1 innerhalb des Gehäuses 16 hervor. Das proximale Ende des inneren Schlauchs 4 bildet mit der Hülse 84 einen Anschlag für den Druckkolben 7. In den Figuren 6 bis 9 und 13 und 14 liegt eine distale Pressfläche 86 des Druckkolbens 7 flächenbündig an diesem Anschlag an, so dass die gesamte Flüssigkeit oder das gesamte Gas im Innenraum des inneren Schlauchs 4 und in dem Indikator enthalten ist. Die Flüssigkeit oder das Gas, die auch als ein zweites Fluid aufgefasst werden können, drücken dabei auf die Innenwandung des inneren Schlauchs 4 und halten diesen so im expandierten Zustand. Der Druckkolben 7 ist mit zwei umlaufenden Dichtungen 88 gegen den Hubraum im Gehäuse 16 abgedichtet, so dass kein Gas oder keine Flüssigkeit zwischen dem Druckkolben 7 und der Innenwand des Gehäuses 16 entweichen kann. Am proximalen Ende (in den Figuren 1 bis 4 und 6 und 7 oben, in den Figuren 8, 11 und 13 oben links, in den Figuren 9, 10, 12 und 14 links) des Druckkolbens 7 ist ein Griffstück 90 angeordnet, mit dem der Druckkolben 7 manuell in das Gehäuse 16 hinein gedrückt und aus dem Gehäuse 16 heraus gezogen werden kann, sofern der Druckkolben 7 nicht mit der Arretierung 14 arretiert ist.

Der Konnektor 6 mündet zwischen der Hülse 84 und dem proximalen Ende 80 des äußeren Schlauchs 1 in das Gehäuse 16. An dieser Stelle begrenzt das Gehäuse 16 in seinem Inneren zusammen mit dem inneren Schlauch 4, der Hülse 84 und dem äußeren Schlauch 1 einen Hohlraum 92, der auf seiner proximalen Seite von der Hülse 84 begrenzt ist und auf seiner distalen Seite im Wesentlichen von dem proximalen Ende 80 des äußeren Schlauchs 1 begrenzt ist. Das Fluid 97 kann ausgehend von diesem Hohlraum 92 in distaler Richtung in den Zwischenraum zwischen den inneren Schlauch 4 und den äußeren Schlauch 1 gepresst werden, wenn sich der innere Schlauch 4 im radial kontrahierten Zustand befindet.

Der Druckindikator ist in seinem inneren über einen Kanal 96 mit dem Innenraum des inneren Schlauchs 4 verbunden. Der Stift 8 ist dazu in einer Kammer des Gehäuses 9 des Stifts 8 angeordnet, wobei die Kammer mit dem Kanal 96 für die Flüssigkeit oder das Gas durchlässig verbunden ist. Der Stift 8 ist mit einer Feder 94 so gelagert, dass er aus dem Gehäuse 9 für den Stift 8 herausgedrückt wird. Der Stift 8 ist dabei gegen das Gehäuse 9 des Stifts 8 abgedichtet. Im radial kontrahierten Zustand des inneren Schlauchs 4 wird der Stift 8 gegen die Kraft der Feder 94 in das Gehäuse 9 hineingezogen. Ansonsten steht der Stift 8 aus dem Gehäuse 9 vor. So ist von außen leicht erkennbar, ob sich der innere Schlauch 4 im kontrahierten oder im expandierten Zustand befindet. Hierdurch wird eine mögliche Fehlfunktion des Druckkolbens 7 und damit der Vorrichtung für den Anwender erkennbar.

Das Fluid 97 kann über eine Spritze 98 mit Hilfe eines Spritzenkolbens 99 in den Konnektor 6 eingespritzt werden. Hierzu wird die Spritze 98 enthaltend das Fluid 97 an den Anschlussadapter 12 angeschlossen. Durch den mit dem Spritzenkolben 99 auf das Fluid 97 ausgeübten Druck wird das Rückschlagventil 82 gegen dessen Druckfeder geöffnet und das Fluid 97 strömt durch den Konnektor 6 in den Hohlraum 92 im Gehäuse 16. Da sich der innere Schlauch 4 im expandierten Zustand befindet, dichtet er den äußeren Schlauch 1 gegen den inneren Schlauch 4 ab (siehe Figur 8). Dadurch kann das Fluid 97 zunächst nicht zwischen den äußeren Schlauch 1 und den expandierten inneren Schlauch 4 vordringen.

Zunächst wird die Arretierung 14 durch Herausziehen der Arretierung 14 aus dem Gehäuse 15 von dem Druckkolben 7 gelöst (siehe Figur 9). Danach wird der Druckkolben 7 mit dem Griff 90 in proximaler Richtung gezogen, um den Zwischenraum zwischen dem äußeren Schlauch 1 und dem inneren Schlauch 4 sowie die durchgehenden Öffnungen 2 in der Schlauchwandung des äußeren Schlauchs 1 zu öffnen. Dadurch strömt Gas oder Flüssigkeit aus dem Inneren des inneren Schlauchs 4 in den Hubraum des Druckkolbens 7 im Inneren des Gehäuses 16. Der innere Schlauch 4 wird so in den radial kontrahierten Zustand überführt und dabei die Öffnungen 2 und der Zwischenraum zwischen dem äußeren Schlauch 1 und dem inneren Schlauch 4 vom Hohlraum 92 aus geöffnet. Der Druckkolben 7 wird durch Einschieben der Arretierung 14 in das Gehäuse 15 arretiert, damit der Druckkolben 7 nicht aufgrund der in dem inneren Schlauch 4 durch dessen Vorformung gespeicherten elastischen Kraft wieder in den Hubraum des Gehäuses 16 gezogen wird (siehe Figur 10). Mit der Arretierung 14 wird also verhindert, dass sich der radial kontrahierte Zustand wieder von alleine in den entspannten expandierten Zustand überführt. Theoretisch ist es auch möglich, den radial kontrahierten Zustand als entspannten Zustand zu verwenden. Es wird jedoch bevorzugt, dass der expandierte Zustand der entspannte Zustand ist, weil so bei einer Undichtigkeit des Hubraums, des inneren Schlauchs 4 oder des Indikators die Öffnungen 2 geschlossen sind oder werden. Der so erreichte radial kontrahierte Zustand des inneren Schlauchs 4 lässt sich daran erkennen, dass der Stift 8 in das Gehäuse 9 des Stifts 8 eingezogen beziehungsweise versenkt wird.

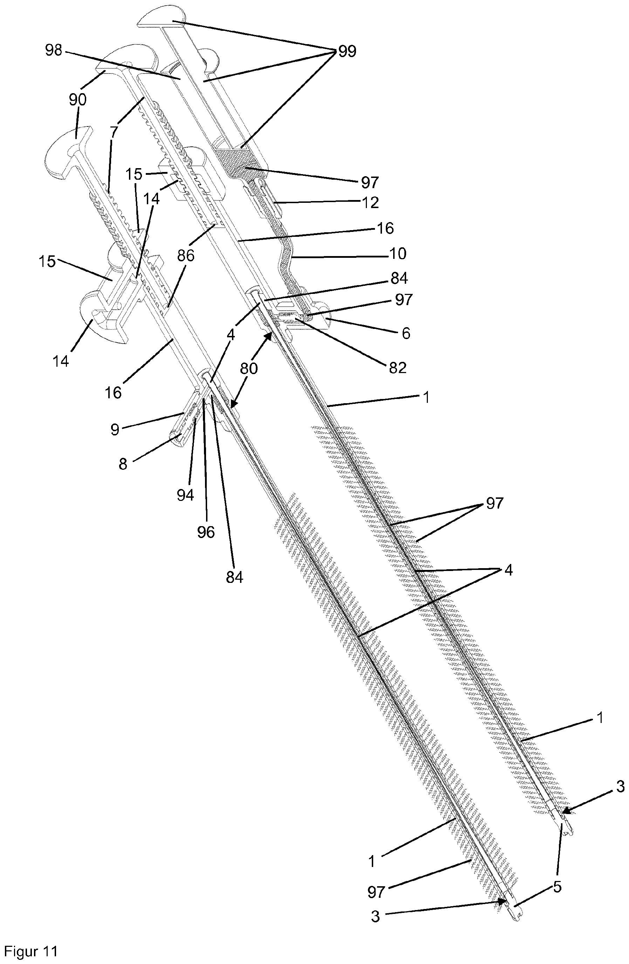

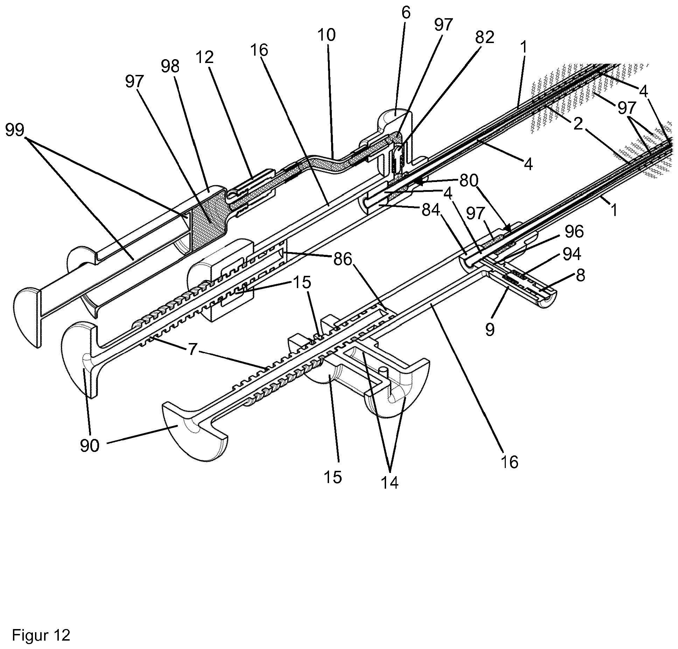

Nach dem Öffnen des Zwischenraums und der Öffnungen 2 wird mit der Spritze das Fluid 97 durch den Zwischenraum zwischen dem äußeren Schlauch 1 und dem inneren Schlauch 4 gepresst und von dort aus den Öffnungen 2 herausgedrückt (siehe die Figuren 11 und 12). Die Summe der freien Querschnitte der Öffnungen 2 ist kleiner als der freie Querschnitt des Zwischenraums, damit auch bei den am weitesten distal angeordneten Öffnungen 2, die im Bereich des Verbindungselements 5 liegen, noch ein ausreichender Druck des Fluids 97 vorhanden ist, so dass auch in diesem Bereich der gewünschte Austrag des Fluids 97 stattfindet. Dementsprechend müssen die einzelnen Öffnungen 2 aufgrund ihrer großen Anzahl (bevorzugt mehr als hundert) sehr kleine freie Querschnitte haben. Beispielsweise können die Öffnungen 2 bei einem Innendurchmesser des inneren Schlauchs von etwa 5 mm und einem Außendurchmesser des inneren Schlauchs 4 von etwa 1,5 mm kreisrunde durchgehende zylindrische Löcher mit einem Durchmesser zwischen 10 µm bis 50 µm sein.

Nach dem Einleiten einer gewünschten Menge des Fluids 97 durch die Öffnungen 2 werden der Zwischenraum und die Öffnungen 2 wieder durch Expandieren des inneren Schlauchs 4 geschlossen, indem die Arretierung 14 gelöst wird und der Druckkolben 7 wieder in das Gehäuse 16 eingeschoben wird. Die noch im Zwischenraum zwischen dem äußeren Schlauch 1 und dem inneren Schlauch 4 befindlichen Anteile des Fluids werden dabei durch die Öffnungen 2 herausgedrückt. Dieser Vorgang kann solange wiederholt werden, bis die Spritze kein Fluid 97 mehr enthält. Dann muss die Spritze gegebenenfalls gewechselt werden.

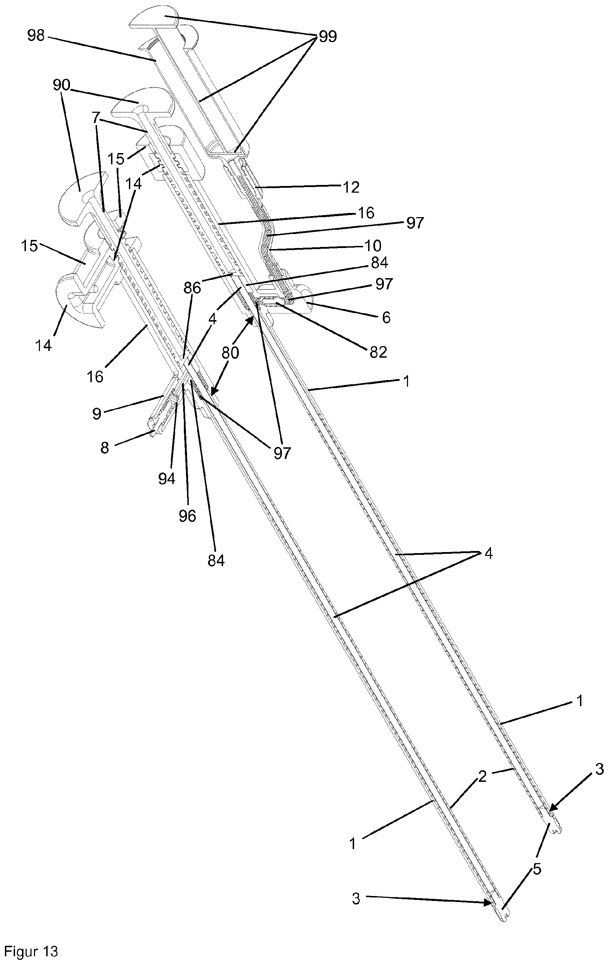

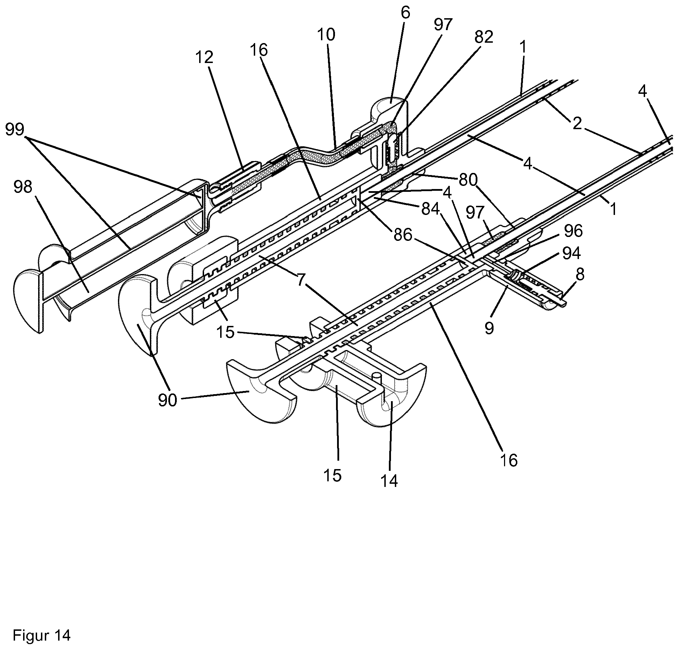

Wenn also die Spritze leer ist, wird der innere Schlauch 4 wieder in den expandierten Zustand überführt (siehe Figuren 13 und 14). Die Spritze kann dann entfernt werden oder, wenn dies gewünscht ist, durch eine volle Spritze mit dem gleichen Fluid 97 oder einem anderen Fluid ersetzt werden. Auf diese Weise ist eine Variation einer Behandlung oder eines Spülvorgangs möglich.

Beim Wechsel vom radial kontrahierten Zustand in den expandierten Zustand und umgekehrt wird der äußere Schlauch 1 nur einer sehr geringen Kraft ausgesetzt, so dass dieser in seiner äußeren Form und insbesondere in seiner Länge weitgehend oder vollständig unverändert bleibt. Hierdurch erfolgt das Spülen an den gewünschten Stellen. Bei einer Implantation des Schlauchsystems ist angrenzendes Gewebe keiner mechanischen Belastung ausgesetzt, so dass eine Reizung des entzündeten Gewebes vermieden werden kann. Zudem wird durch das Schließen der Öffnungen 2 vermieden, dass Gewebe bei einer längeren Verweildauer in den Zwischenraum zwischen dem äußeren Schlauch 1 und dem inneren Schlauch 4 einwachsen kann, so in Hinterschneidungen greift und es dadurch beim Entfernen des Schlauchsystems zu Verletzungen oder unerwünschten Irritationen des gerade behandelten Gewebes kommt.

Vor der Anwendung der Vorrichtung kann diese durch geeignetes plastisches Verformen des Schlauchsystems mit dem äußeren Schlauch 1 und dem inneren Schlauch 4 an den zu spülenden Hohlraum angepasst werden. Das Schlauchsystem und die Vorrichtung zeichnen sich aber auch insbesondere dadurch aus, dass die Länge des Schlauchsystem auf einfache Weise an den zu spülenden oder zu behandelnden Hohlraum angepasst werden kann. Das Schlauchsystem kann vor dem Einsatz einfach bei der gewünschten Länge auf der distalen Seite abgeschnitten werden. Das Verbindungselement 5 wird aus dem abgeschnittenen Teil des inneren Schlauchs 4 herausgeschraubt und in das durch den Schnitt entstandene distale Ende des inneren Schlauchs 4 wieder eingeschraubt. Dadurch werden der innere Schlauch 4 und der äußere Schlauch 1 an deren distalen Ende wieder dicht verschlossen und miteinander verbunden.

Die erfindungsgemäßen Vorrichtungen werden so verwendet, dass zur Applikation von Fluiden 97 zuerst die Flüssigkeit oder das Gas aus dem inneren Schlauch 4 durch Betätigung der Vorrichtung zur Erzeugung von Unterdruck, das heißt, durch Herausziehen des Druckkolbens 7, 27, 47, 67 aus dem Gehäuse 16, 36, 56, 76 zumindest teilweise entfernt wird. Dabei kollabiert der innere Schlauch 4 und es entsteht ein Hohlraum zwischen der Außenseite des inneren Schlauchs 4 und der Innenseite des äußeren Schlauchs 1, 21, 41, 61. Die mindestens eine Öffnung 2, 22, 42, 62 in der Mantelfläche des äußeren Schlauchs 1, 21, 41, 61 wird freigelegt. Dann wird über den Konnektor 6, 26, 46, 66 in den Zwischenraum zwischen dem inneren Schlauch 4 und dem äußeren Schlauch 1, 21, 41, 61 das zu applizierende Fluid 97 eingepresst. Vorteilhaft kann dabei das Einpressen mit einer üblichen Kunststoffspritze erfolgen. Das zu applizierende Fluid 97 fließt durch den Zwischenraum und danach tritt das Fluid 97 durch die Öffnungen 2, 22, 42, 62 des äußeren Schlauchs 1, 21, 41, 61 in die Umgebung aus. Nach erfolgter Applikation einer ausreichenden Menge des Fluids 97 wird die Vorrichtung zur Erzeugung von Unterdruck (der Druckkolben 7) zurückgestellt, so dass ein Gleichgewicht zwischen dem Außendruck der Umgebung und dem Innendruck der Flüssigkeit oder des Gases im inneren Schlauch 4 ausgebildet wird. Der elastische innere Schlauch 4 relaxiert infolge seiner Rückstellkraft in seine Ausgangsform und verschließt die Öffnungen 2, 22, 42, 62 im äußeren Schlauch 1, 21, 41, 61. Dieser Prozess kann im Prinzip beliebig oft widerholt werden.

Das Verbindungselement 5, 25, 45, 65 kann nach Kürzen der Vorrichtung vom medizinischen Anwender einfach in das distale (erste) Schlauchende des inneren Schlauchs 4 eingeschraubt oder eingesteckt werden. Dadurch wird einerseits der innere Schlauch 4 verschlossen und gleichzeitig presst der durch das Verbindungselement 5, 25, 45, 65 expandierte innere Schlauch 4 gegen die Innenwand des äußeren Schlauchs 1, 21, 41, 61, so dass der innere Schlauch 4 gegen den äußeren Schlauch 1, 21, 41, 61 festgeklemmt wird und der Zwischenraum zwischen dem inneren Schlauch 4 und dem äußeren Schlauch 1, 21, 41, 61 verschlossen wird.

Als das zu applizierendes Fluid 97 kann je nach Anwendung beispielsweise eine desinfizierende Flüssigkeit oder eine wässrige Lösung umfassend wenigstens ein Antibiotikum verwendet werden.

Für eine medizinische Anwendung der Vorrichtungen können die Schläuche 1, 4, 21, 41, 61 und vorzugsweise auch die Verbindungselemente 5, 25, 45, 65 aus biokompatiblen Materialien aufgebaut werden, in denen Röntgenopaker enthalten sind, so dass die Lage des äußeren Schlauchs 1, 21, 41, 61 und gegebenenfalls des Verbindungselements 5, 25, 45, 65 mit bildgebenden Röntgenverfahren bestimmbar ist.

### Bezugszeichenliste

- 1, 21, 41, 61: Äußerer Schlauch
- 2, 22, 42, 62: Öffnung
- 3, 23, 43, 63: Erstes (distales) Ende
- 4: Innerer Schlauch
- 5, 25, 45, 65: Verbindungselement
- 6, 26, 46, 66: Konnektor
- 7, 27, 47, 67: Druckkolben
- 8: Stift
- 9: Gehäuse für Stift
- 10, 30, 50, 70: Leitung
- 12, 32, 52, 72: Anschlussadapter
- 14, 34, 54, 74: Arretierung
- 15, 35, 55, 75: Gehäuse für Arretierung
- 16, 36, 56, 76: Gehäuse für Druckkolben
- 48: Membran
- 49: Anschluss für die Membran
- 68: Ballon
- 69: Anschluss für Ballon
- 80: zweites (proximales) Ende
- 82: Rückschlagventil
- 84: Hülse
- 86: Pressfläche des Druckkolbens
- 88: Dichtungsring
- 90: Griffstück
- 92: Hohlraum
- 94: Feder
- 96: Kanal
- 97: Fluid
- 98: Spritze
- 99: Spritzenkolben

## Patentansprüche

1. Medizinische Vorrichtung zur lokalen Applikation eines Fluids (97) aufweisend einen flexibel verformbaren äußeren Schlauch (1, 21, 41, 61) mit einer Schlauchwand, wobei der äußere Schlauch (1, 21, 41, 61) mehrere durchgehende Öffnungen (2, 22, 42, 62) in der Schlauchwand aufweist, wobei zumindest eine der mehreren durchgehenden Öffnungen (2, 22, 42, 62) im Bereich eines ersten Endes (3, 23, 43, 63) des äußeren Schlauchs (1, 21, 41, 61) angeordnet ist,
einen flexiblen inneren Schlauch (4), wobei der innere Schlauch (4) zumindest bereichsweise im Inneren des äußeren Schlauchs (1, 21, 41, 61) angeordnet ist, wobei der innere Schlauch (4) in einem expandierten Zustand einen Außendurchmesser hat, der zumindest genauso groß ist, wie der Innendurchmesser des äußeren Schlauchs (1, 21, 41, 61), so dass der innere Schlauch (4) die durchgehenden Öffnungen (2, 22, 42, 62) des äußeren Schlauchs (1, 21, 41, 61) im expandierten Zustand an der Innenseite des äußeren Schlauchs (1, 21, 41, 61) verschließt, wobei der Außendurchmesser des inneren Schlauchs (4) von dem expandierten Zustand in einen radial kontrahierten Zustand mit geringerem Außendurchmesser des inneren Schlauchs (4) überführbar ist, so dass die Öffnungen (2, 22, 42, 62) freigelegt sind,
eine Verbindung (5, 25, 45, 65), die den äußeren Schlauch (1, 21, 41, 61) und den inneren Schlauch (4) am ersten Ende (3, 23, 43, 63) des äußeren Schlauchs (1, 21, 41, 61) fest miteinander verbindet und fluiddicht verschließt, und
einen Konnektor (6, 26, 46, 66) zum Einspeisen des Fluids (97) in den Zwischenraum zwischen der Innenseite des äußeren Schlauchs (1, 21, 41, 61) und der Außenseite des inneren Schlauchs (4), wobei der Konnektor (6, 26, 46, 66) im Bereich eines zweiten Endes (80) des äußeren Schlauchs (1, 21, 41, 61) angeordnet ist, wobei das zweite Ende des äußeren Schlauchs (1, 21, 41, 61) dem ersten Ende (3, 23, 43, 63) des äußeren Schlauchs (1, 21, 41, 61) gegenüberliegend angeordnet ist,
**dadurch gekennzeichnet, dass**
der expandierte Zustand der entspannte Zustand des inneren Schlauchs (4) ist und durch eine Krafteinwirkung in den radial kontrahierten Zustand überführbar ist.

2. Vorrichtung nach Anspruch 1, **dadurch gekennzeichnet, dass**
der innere Schlauch (4) durch Erzeugen eines Unterdrucks im Inneren des inneren Schlauchs (4) oder durch eine Volumenreduktion einer Flüssigkeit im Inneren des inneren Schlauchs (4) in den radial kontrahierten Zustand überführbar ist.

3. Vorrichtung nach Anspruch 1 oder 2, **dadurch gekennzeichnet, dass**
der innere Schlauch (4) durch einen Verschluss des äußeren Schlauchs (1, 21, 41, 61) am zweiten Ende (80) des äußeren Schlauchs (1, 21, 41, 61) oder durch die seitliche Schlauchwand des äußeren Schlauchs (1, 21, 41, 61) im Bereich des zweiten Endes (80) geführt ist.

4. Vorrichtung nach einem der vorangehenden Ansprüche, **dadurch gekennzeichnet, dass**
die Vorrichtung eine Bedieneinrichtung aufweist, insbesondere eine Kolbenspritze, eine Pumpe oder einen Peleusball als Bedieneinrichtung aufweist, mit der der innere Schlauch (4) innerhalb des äußeren Schlauchs (1, 21, 41, 61) von dem expandierten Zustand in den radial kontrahierten Zustand überführbar ist, so dass die Öffnungen (2, 22, 42, 62) im äußeren Schlauch (1, 21, 41, 61) geöffnet werden.

5. Vorrichtung nach einem der vorangehenden Ansprüche, **dadurch gekennzeichnet, dass**
die Verbindung (5, 25, 45, 65) durch ein Verbindungselement (5, 25, 45, 65) realisiert ist.

6. Vorrichtung nach einem der vorangehenden Ansprüche, **dadurch gekennzeichnet, dass**
der Zwischenraum zwischen dem äußeren Schlauch (1, 21, 41, 61) und dem inneren Schlauch (4) am zweiten Ende (80) des äußeren Schlauchs (1, 21, 41, 61) fluiddicht verschlossen ist.

7. Vorrichtung nach einem der vorangehenden Ansprüche, **dadurch gekennzeichnet, dass**
der äußere Schlauch (1, 21, 41, 61) an seinem ersten Ende (3, 23, 43, 63) den inneren Schlauch (4) umschließt.

8. Vorrichtung nach einem der vorangehenden Ansprüche, **dadurch gekennzeichnet, dass**
ein Gas in dem inneren Schlauch (4) enthalten ist, wobei der innere Schlauch (4) durch Reduzieren des Drucks des Gases im inneren Schlauch (4) in den radial kontrahierten Zustand überführbar ist und durch erneutes Einleiten des Gases oder eines Gases wieder in den expandierten Zustand überführbar ist, oder
eine Flüssigkeit den inneren Schlauch (4) füllt, wobei der innere Schlauch (4) durch Absaugen eines Teils der Flüssigkeit aus dem inneren Schlauch (4) in den radial kontrahierten Zustand überführbar ist und durch erneutes Einleiten der Flüssigkeit oder anderer Flüssigkeit in den inneren Schlauch (4) wieder in den expandierten Zustand überführbar ist.

9. Vorrichtung nach einem der vorangehenden Ansprüche, **dadurch gekennzeichnet, dass**
die Innenseite des äußeren Schlauchs (1, 21, 41, 61) mit der Außenseite des inneren Schlauchs (4) ein Ventil bildet.

10. Vorrichtung nach einem der vorangehenden Ansprüche, **dadurch gekennzeichnet, dass**
die mehreren durchgehenden Öffnungen (2, 22, 42, 62) alle, paarweise oder gruppenweise in axialer Richtung des äußeren Schlauchs (1, 21, 41, 61) zueinander beabstandet sind.

11. Vorrichtung nach einem der vorangehenden Ansprüche, **dadurch gekennzeichnet, dass**
im Inneren des inneren Schlauchs (4) Wasser, eine physiologische Kochsalzlösung oder eine Ringer-Lösung oder Luft angeordnet ist, wobei durch eine Verringerung des Drucks der Luft oder des Volumens des Wassers, der physiologischen Kochsalzlösung oder der Ringer-Lösung der innere Schlauch (4) in den radial kontrahierten Zustand überführbar ist.

12. Vorrichtung nach einem der vorangehenden Ansprüche, **dadurch gekennzeichnet, dass**
ein Indikator mit dem inneren Schlauch (4) verbunden ist, an dem von außerhalb der Vorrichtung ablesbar ist, ob sich der innere Schlauch (4) im expandierten Zustand oder im kontrahierten Zustand befindet, wobei vorzugsweise der Indikator ein Unterdruckindikator ist, der mit dem inneren Schlauch (4) gasdurchlässig verbunden ist.

13. Vorrichtung nach einem der vorangehenden Ansprüche, **dadurch gekennzeichnet, dass**
in der Wandung des inneren Schlauchs (4) zumindest ein Metalldraht oder zumindest eine Metallspirale angeordnet ist, wobei der zumindest eine Metalldraht oder die zumindest eine Metallspirale bevorzugt entlang der gesamten Länge des inneren Schlauchs (4) angeordnet sind.

14. Vorrichtung nach einem der vorangehenden Ansprüche, **dadurch gekennzeichnet, dass**
in dem Konnektor (6, 26, 46, 66) oder in einer Verbindung des Konnektors (6, 26, 46, 66) zu dem Zwischenraum zwischen dem äußeren Schlauch (1, 21, 41, 61) und dem inneren Schlauch (4) ein Rückschlagventil (82) angeordnet ist, das ein Fließen des Fluids (97) aus dem Zwischenraum heraus in den Konnektor (6, 26, 46, 66) hinein oder aus dem Konnektor (6, 26, 46, 66) heraus verhindert.

15. Schlauchsystem zum Aufbau einer Vorrichtung nach einem der vorangehenden Ansprüche, **gekennzeichnet durch**
einen flexibel verformbaren äußeren Schlauch (1, 21, 41, 61) mit einer Schlauchwand, wobei der äußere Schlauch (1, 21, 41, 61) mehrere durchgehende Öffnungen (2, 22, 42, 62) in der Schlauchwand aufweist,
einen flexiblen inneren Schlauch (4), wobei der innere Schlauch (4) zumindest bereichsweise im Inneren des äußeren Schlauchs (1, 21, 41, 61) angeordnet ist, wobei der innere Schlauch (4) in einem expandierten Zustand einen Außendurchmesser hat, der zumindest genauso groß ist, wie der Innendurchmesser des äußeren Schlauchs (1, 21, 41, 61), so dass der innere Schlauch (4) die durchgehenden Öffnungen (2, 22, 42, 62) des äußeren Schlauchs (1, 21, 41, 61) im expandierten Zustand an der Innenseite des äußeren Schlauchs (1, 21, 41, 61) verschließt, wobei der Außendurchmesser des inneren Schlauchs (4) von dem expandierten Zustand in einen radial kontrahierten Zustand mit geringerem Außendurchmesser des inneren Schlauchs (4) überführbar ist, so dass die Öffnungen (2, 22, 42, 62) freigelegt sind,
**dadurch gekennzeichnet, dass**
der expandierte Zustand der entspannte Zustand des inneren Schlauchs (4) ist und durch eine Krafteinwirkung in den radial kontrahierten Zustand überführbar ist.

16. Verfahren zum Betreiben einer Vorrichtung nach einem der Ansprüche 1 bis 14 umfassend die folgenden Schritte
A) Überführen des inneren Schlauchs (4) vom expandierten Zustand in den kontrahierten Zustand, wobei dadurch die Öffnungen (2, 22, 42, 62) in dem äußeren Schlauch (1, 21, 41, 61) geöffnet werden;
B) Einleiten des Fluids (97) in den Zwischenraum zwischen dem äußeren Schlauch (1, 21, 41, 61) und dem inneren Schlauch (4).

## Claims

1. A medical apparatus for local application of a fluid (97), comprising
a flexibly deformable outer tube (1, 21, 41, 61) having a tube wall, the outer tube (1, 21, 41, 61) having a plurality of continuous openings (2, 22, 42, 62) in the tube wall, at least one of the plurality of continuous openings (2, 22, 42, 62) being arranged in the region of a first end (3, 23, 43, 63) of the outer tube (1, 21, 41, 61),
a flexible inner tube (4), the inner tube (4) being arranged at least in regions inside the outer tube (1, 21, 41, 61), the inner tube (4), in an expanded state, having an outer diameter which is at least as large as the inner diameter of the outer tube (1, 21, 41, 61), such that the inner tube (4) closes the continuous openings (2, 22, 42, 62) in the outer tube (1, 21, 41, 61) in the expanded state on the inside of the outer tube (1, 21, 41, 61), the outer diameter of the inner tube (4) being transferable from the expanded state to a radially contracted state with a smaller outer diameter of the inner tube (4), such that the openings (2, 22, 42, 62) are exposed,
a connection (5, 25, 45, 65) rigidly interconnecting and fluid-tightly closing the outer tube (1, 21, 41, 61) and the inner tube (4) at the first end (3, 23, 43, 63) of the outer tube (1, 21, 41, 61), and
a connector (6, 26, 46, 66) for feeding the fluid (97) into the space between the inside of the outer tube (1, 21, 41, 61) and the outside of the inner tube (4), the connector (6, 26, 46, 66) being arranged in the region of a second end (80) of the outer tube (1, 21, 41, 61), the second end of the outer tube (1, 21, 41, 61) being arranged opposite the first end (3, 23, 43, 63) of the outer tube (1, 21, 41, 61),
**characterized in that**
the expanded state is the relaxed state of the inner tube (4) and can be transferred to the radially contracted state by means of application of force.

2. The apparatus according to claim 1, **characterized in that**
the inner tube (4) can be transferred to the radially contracted state by generating a negative pressure inside the inner tube (4) or by a volume reduction of a liquid inside the inner tube (4).

3. The apparatus according to claim 1 or claim 2, **characterized in that**
the inner tube (4) is guided through a closure of the outer tube (1, 21, 41, 61) at the second end (80) of the outer tube (1, 21, 41, 61) or through the lateral tube wall of the outer tube (1, 21, 41, 61) in the region of the second end (80).

4. The apparatus according to any of the preceding claims, **characterized in that**
the apparatus comprises an operating device, in particular comprises a piston syringe, a pump or a pressure ball as the operating device, by means of which the inner tube (4) within the outer tube (1, 21, 41, 61) can be transferred from the expanded state to the radially contracted state, so that the openings (2, 22, 42, 62) in the outer tube (1, 21, 41, 61) are opened.

5. The apparatus according to any of the preceding claims, **characterized in that**
the connection (5, 25, 45, 65) is provided by a connecting element (5, 25, 45, 65).

6. The apparatus according to any of the preceding claims, **characterized in that**
the space between the outer tube (1, 21, 41, 61) and the inner tube (4) is fluid-tightly closed at the second end (80) of the outer tube (1, 21, 41, 61).

7. The apparatus according to any of the preceding claims, **characterized in that**
the outer tube (1, 21, 41, 61) encloses the inner tube (4) at its first end (3, 23, 43, 63).

8. The apparatus according to any of the preceding claims, **characterized in that**
a gas is contained in the inner tube (4), the inner tube (4) being transferable to the radially contracted state by reducing the pressure of the gas in the inner tube (4) and being transferable back to the expanded state by re-introducing the gas or a gas, or
a liquid fills the inner tube (4), the inner tube (4) being transferable to the radially contracted state by sucking a portion of the liquid out of the inner tube (4) and being transferable back to the expanded state by re-introducing the liquid or other liquid into the inner tube (4).

9. The apparatus according to any of the preceding claims, **characterized in that**
the inside of the outer tube (1, 21, 41, 61) forms a valve with the outer side of the inner tube (4).

10. The apparatus according to any of the preceding claims, **characterized in that**
the plurality of continuous openings (2, 22, 42, 62) are all spaced apart from one another in pairs or in groups in the axial direction of the outer tube (1, 21, 41, 61).

11. The apparatus according to any of the preceding claims, **characterized in that**
water, a physiological saline solution or a Ringer's solution or air is arranged inside the inner tube (4), the inner tube (4) being transferable to the radially contracted state by reducing the pressure of the air or the volume of the water, the physiological saline solution or the Ringer's solution.

12. The apparatus according to any of the preceding claims, **characterized in that**
an indicator is connected to the inner tube (4), on which indicator it can be read from outside the apparatus whether the inner tube (4) is in the expanded state or in the contracted state, the indicator preferably being a negative-pressure indicator connected to the inner tube (4) in a gas-permeable manner.

13. The apparatus according to any of the preceding claims, **characterized in that**
at least one metal wire or at least one metal coil is arranged in the wall of the inner tube (4), the at least one metal wire or the at least one metal coil preferably being arranged along the entire length of the inner tube (4).

14. The apparatus according to any of the preceding claims, **characterized in that**
a check valve (82) is arranged in the connector (6, 26, 46, 66) or in a connection of the connector (6, 26, 46, 66) to the space between the outer tube (1, 21, 41, 61) and the inner tube (4), which check valve prevents the fluid (97) from flowing out of the space into the connector (6, 26, 46, 66) or out of the connector (6, 26, 46, 66).

15. A tube system for constructing an apparatus according to any of the preceding claims, **characterized by**
a flexibly deformable outer tube (1, 21, 41, 61) having a tube wall, the outer tube (1, 21, 41, 61) having a plurality of continuous openings (2, 22, 42, 62) in the tube wall,
a flexible inner tube (4), the inner tube (4) being arranged at least in regions inside the outer tube (1, 21, 41, 61), the inner tube (4), in an expanded state, having an outer diameter which is at least as large as the inner diameter of the outer tube (1, 21, 41, 61), such that the inner tube (4) closes the continuous openings (2, 22, 42, 62) of the outer tube (1, 21, 41, 61) in the expanded state on the inside of the outer tube (1, 21, 41, 61), the outer diameter of the inner tube (4) being transferable from the expanded state to a radially contracted state with a smaller outer diameter of the inner tube (4), such that the openings (2, 22, 42, 62) are exposed,
**characterized in that**
the expanded state is the relaxed state of the inner tube (4) and can be transferred to the radially contracted state by means of application of force.

16. A method for operating an apparatus according to any of claims 1 to 14, comprising the following steps
A) transferring the inner tube (4) from the expanded state to the contracted state, thereby opening the openings (2, 22, 42, 62) in the outer tube (1, 21, 41, 61);
B) introducing the fluid (97) into the space between the outer tube (1, 21, 41, 61) and the inner tube (4).

## Revendications

1. Dispositif médical permettant l'application locale d'un fluide (97), présentant
un tuyau extérieur (1, 21, 41, 61) déformable de manière flexible, comportant une paroi de tuyau, dans lequel le tuyau extérieur (1, 21, 41, 61) présente multiples ouvertures traversantes (2, 22, 42, 62) dans la paroi de tuyau, dans lequel au moins l'une des multiples ouvertures traversantes (2, 22, 42, 62) est disposée dans la région d'une première extrémité (3, 23, 43, 63) du tuyau extérieur (1, 21, 41, 61),
un tuyau intérieur (4) flexible, dans lequel le tuyau intérieur (4) est disposé, au moins dans certaines régions, à l'intérieur du tuyau extérieur (1, 21, 41, 61), dans lequel, dans un état expansé, le tuyau intérieur (4) possède un diamètre externe qui est au moins aussi grand que le diamètre interne du tuyau extérieur (1, 21, 41, 61), de sorte que le tuyau intérieur (4) obture les ouvertures traversantes (2, 22, 42, 62) du tuyau extérieur (1, 21, 41, 61) dans l'état expansé au niveau de la face interne du tuyau extérieur (1, 21, 41, 61), dans lequel le diamètre externe du tuyau intérieur (4) peut être transféré de l'état expansé vers un état radialement contracté comportant un diamètre externe réduit du tuyau intérieur (4), de sorte que les ouvertures (2, 22, 42, 62) sont exposées,
un lien (5, 25, 45, 65) qui relie de manière fixe le tuyau extérieur (1, 21, 41, 61) et le tuyau intérieur (4) l'un à l'autre au niveau de la première extrémité (3, 23, 43, 63) du tuyau extérieur (1, 21, 41, 61) et les obture de manière étanche aux fluides, et
un connecteur (6, 26, 46, 66) permettant d'introduire le fluide (97) dans l'espace intermédiaire entre la face interne du tuyau extérieur (1, 21, 41, 61) et la face externe du tuyau intérieur (4), dans lequel le connecteur (6, 26, 46, 66) est disposé dans la région d'une seconde extrémité (80) du tuyau extérieur (1, 21, 41, 61), dans lequel la seconde extrémité du tuyau extérieur (1, 21, 41, 61) est disposée à l'opposé de la première extrémité (3, 23, 43, 63) du tuyau extérieur (1, 21, 41, 61),
**caractérisé en ce que**
l'état expansé est l'état détendu du tuyau intérieur (4) et peut être transféré vers l'état radialement contracté sous l'effet d'une force.

2. Dispositif selon la revendication 1, **caractérisé en ce que**
le tuyau intérieur (4) peut être transféré vers l'état radialement contracté par la génération d'une pression négative à l'intérieur du tuyau intérieur (4) ou par une réduction de volume d'un liquide à l'intérieur du tuyau intérieur (4).

3. Dispositif selon la revendication 1 ou 2, **caractérisé en ce que**
le tuyau intérieur (4) est guidé à travers un obturateur du tuyau extérieur (1, 21, 41, 61) au niveau de la seconde extrémité (80) du tuyau extérieur (1, 21, 41, 61) ou à travers la paroi de tuyau latérale du tuyau extérieur (1, 21, 41, 61) dans la région de la seconde extrémité (80).

4. Dispositif selon l'une des revendications précédentes, **caractérisé en ce que**
le dispositif présente un appareil de commande, en particulier une seringue à piston, une pompe ou une poire aspirante comme appareil de commande, au moyen duquel le tuyau intérieur (4) à l'intérieur du tuyau extérieur (1, 21, 41, 61) peut être transféré de l'état expansé vers l'état radialement contracté, de sorte que les ouvertures (2, 22, 42, 62) dans le tuyau extérieur (1, 21, 41, 61) sont ouvertes.

5. Dispositif selon l'une des revendications précédentes, **caractérisé en ce que**
le lien (5, 25, 45, 65) est réalisé par un élément de liaison (5, 25, 45, 65).

6. Dispositif selon l'une des revendications précédentes, **caractérisé en ce que**
l'espace intermédiaire entre le tuyau extérieur (1, 21, 41, 61) et le tuyau intérieur (4) est obturé de manière étanche aux fluides au niveau de la seconde extrémité (80) du tuyau extérieur (1, 21, 41, 61).

7. Dispositif selon l'une des revendications précédentes, **caractérisé en ce que**
le tuyau extérieur (1, 21, 41, 61) entoure le tuyau intérieur (4) au niveau de sa première extrémité (3, 23, 43, 63).

8. Dispositif selon l'une des revendications précédentes, **caractérisé en ce que**
un gaz est contenu dans le tuyau intérieur (4), dans lequel le tuyau intérieur (4) peut être transféré vers l'état radialement contracté par la réduction de la pression du gaz dans le tuyau intérieur (4) et peut être transféré de nouveau vers l'état expansé par une réintroduction du gaz ou d'un gaz, ou
un liquide remplit le tuyau intérieur (4), dans lequel le tuyau intérieur (4) peut être transféré vers l'état radialement contracté par l'aspiration d'une partie du liquide hors du tuyau intérieur (4) et peut être transféré de nouveau vers l'état expansé par une réintroduction du liquide ou d'un autre liquide dans le tuyau intérieur (4).

9. Dispositif selon l'une des revendications précédentes, **caractérisé en ce que**
la face interne du tuyau extérieur (1, 21, 41, 61) forme une vanne avec la face externe du tuyau intérieur (4).

10. Dispositif selon l'une des revendications précédentes, **caractérisé en ce que**
les multiples ouvertures traversantes (2, 22, 42, 62) sont, toutes, en paire ou en groupe, espacées les unes des autres dans une direction axiale du tuyau extérieur (1, 21, 41, 61).

11. Dispositif selon l'une des revendications précédentes, **caractérisé en ce que**
de l'eau, une solution saline physiologique ou une solution de Ringer ou de l'air est disposé à l'intérieur du tuyau intérieur (4), dans lequel le tuyau intérieur (4) peut être transféré vers l'état radialement contracté par une réduction de la pression de l'air ou du volume de l'eau, de la solution saline physiologique ou de la solution de Ringer.

12. Dispositif selon l'une des revendications précédentes, **caractérisé en ce que**
un indicateur est relié au tuyau intérieur (4), sur lequel indicateur il est possible de lire, depuis l'extérieur du dispositif, si le tuyau intérieur (4) se trouve dans l'état expansé ou dans l'état contracté, dans lequel l'indicateur est de préférence un indicateur de pression négative qui est relié de manière perméable aux gaz au tuyau intérieur (4).

13. Dispositif selon l'une des revendications précédentes, **caractérisé en ce que**
au moins un fil métallique ou au moins une spirale métallique est disposé dans la paroi du tuyau intérieur (4), dans lequel l'au moins un fil métallique ou l'au moins une spirale métallique est disposé de préférence sur toute la longueur du tuyau intérieur (4).

14. Dispositif selon l'une des revendications précédentes, **caractérisé en ce que**
une vanne anti-retour (82) est disposée dans le connecteur (6, 26, 46, 66) ou dans un lien du connecteur (6, 26, 46, 66) vers l'espace intermédiaire entre le tuyau extérieur (1, 21, 41, 61) et le tuyau intérieur (4), laquelle vanne anti-retour empêche un écoulement du fluide (97) hors de l'espace intermédiaire vers le connecteur (6, 26, 46, 66) ou hors du connecteur (6, 26, 46, 66).

15. Système de tuyaux permettant de monter un dispositif selon l'une des revendications précédentes, **caractérisé par**
un tuyau extérieur (1, 21, 41, 61) déformable de manière flexible comportant une paroi de tuyau, dans lequel le tuyau extérieur (1, 21, 41, 61) présente multiples ouvertures traversantes (2, 22, 42, 62) dans la paroi de tuyau,
un tuyau intérieur (4) flexible, dans lequel le tuyau intérieur (4) est disposé, au moins dans certaines régions, à l'intérieur du tuyau extérieur (1, 21, 41, 61), dans lequel, dans un état expansé, le tuyau intérieur (4) possède un diamètre externe qui est au moins aussi grand que le diamètre interne du tuyau extérieur (1, 21, 41, 61), de sorte que le tuyau intérieur (4) obture les ouvertures traversantes (2, 22, 42, 62) du tuyau extérieur (1, 21, 41, 61) dans l'état expansé au niveau de la face interne du tuyau extérieur (1, 21, 41, 61), dans lequel le diamètre externe du tuyau intérieur (4) peut être transféré de l'état expansé vers un état radialement contracté comportant un diamètre externe réduit du tuyau intérieur (4), de sorte que les ouvertures (2, 22, 42, 62) sont exposées,
**caractérisé en ce que**
l'état expansé est l'état détendu du tuyau intérieur (4) et peut être transféré vers l'état radialement contracté sous l'effet d'une force.

16. Procédé permettant de faire fonctionner un dispositif selon l'une des revendications 1 à 14, comprenant les étapes suivantes
A) transfert du tuyau intérieur (4) de l'état expansé vers l'état contracté, dans lequel les ouvertures (2, 22, 42, 62) dans le tuyau extérieur (1, 21, 41, 61) sont ainsi ouvertes ;
B) introduction du fluide (97) dans l'espace intermédiaire entre le tuyau extérieur (1, 21, 41, 61) et le tuyau intérieur (4).
